# EUROPEAN PATENT APPLICATION

(11) **EP 4 675 628 A1**
(43) Date of publication of application: **07.01.2026**
(21) Application number: 25187364.2
(22) Date of filing: 03.07.2025
(51) Int. Cl.: G16B 5/00, G16B 40/00, G01N 15/14, G01N 15/149, G01N 15/10, G01N 15/1429

(54) **METHOD FOR FLOW CYTOMETRY QUALITY SCORES AND SYSTEMS FOR SAME**

(30) Priority: 03.07.2024 US 202463667408 P
(71) Applicant: Becton Dickinson and Company, Franklin Lakes, NJ 07417-1880 (US)
(72) Inventor: Mage, Peter L., Franklin Lakes, 07417-1880 (US); Owsley, Keegan, Franklin Lakes, 07417-1880 (US); Bai, Wenyu, Franklin Lakes, 07417-1880 (US)
(74) Representative: Arnold & Siedsma

(57) **Abstract**

Aspects of the present disclosure include methods for identifying measurement uncertainty associated with light detected from a sample. Methods according to the present disclosure include introducing a sample into a flow cytometer, flowing the introduced sample in a flow stream, irradiating the sample in the flow stream with a light source, detecting light from particles in the sample flowing in the flow stream and, identifying measurement uncertainty associated with the detected light. the present disclosure the present disclosure, measurement uncertainty is identified corresponding to individual particles in the sample. the present disclosure, measurement uncertainty is identified for individual parameters of detected light for particles in the sample. Methods according to the present disclosure further comprise generating a quality score for each particle based on the measurement uncertainty for each particle. Systems, integrated circuit devices (e.g., a field programmable gate array) and non-transitory computer readable storage mediums for practicing the subject methods are also provided.

## Description

This application claims priority to the filing date of United States Provisional Patent Application Serial No. 63/667,408 filed July 3, 2024.

### INTRODUCTION

The characterization of analytes in biological fluids has become an important part of biological research, medical diagnoses and assessments of overall health and wellness of a patient. Detecting analytes in biological fluids, such as human blood or blood derived products, can provide results that may play a role in determining a treatment protocol of a patient having a variety of disease conditions.

Flow cytometry is a technique used to characterize and often times sort biological material, such as cells of a blood sample or particles of interest in another type of biological or chemical sample. A flow cytometer typically includes a sample reservoir for receiving a fluid sample, such as a blood sample, and a sheath reservoir containing a sheath fluid. The flow cytometer transports the particles (including cells) in the fluid sample as a cell stream to a flow cell, while also directing the sheath fluid to the flow cell. To characterize the components of the flow stream, the flow stream is irradiated with light. Variations in the materials in the flow stream, such as morphologies or the presence of fluorescent labels, may cause variations in the observed light and these variations allow for characterization and separation. To characterize the components in the flow stream, light must impinge on the flow stream and be collected. Light sources in flow cytometers can vary and may include one or more broad spectrum lamps, light emitting diodes as well as single wavelength lasers. The light source is aligned with the flow stream and an optical response from the illuminated particles is collected and quantified.

Isolation of biological particles has been achieved by adding a sorting or collection capability to flow cytometers. Particles in a segregated stream, detected as having one or more desired characteristics, are individually isolated from the sample stream by mechanical or electrical removal. A common flow sorting technique utilizes drop sorting in which a fluid stream containing linearly segregated particles is broken into drops. The drops containing particles of interest are electrically charged and deflected into a collection tube by passage through an electric field. Typically, the linearly segregated particles in the stream are characterized as they pass through an observation point situated just below the nozzle tip. Once a particle is identified as meeting one or more desired criteria, the time at which it will reach the drop break-off point and break from the stream in a drop can be predicted. Ideally, a brief charge is applied to the fluid stream just before the drop containing the selected particle breaks from the stream and then grounded immediately after the drop breaks off. The drop to be sorted maintains an electrical charge as it breaks off from the fluid stream, and all other drops are left un-charged.

Flow cytometry is used to measure features of single particles or cells based on optical signals. To effectively identify and/or separate populations of particles or cells based on these measured signals, practitioners must be able to determine whether a measured difference in signal between two particles is due to a true intrinsic difference between those particles or whether the measured difference in signal is due to random measurement error. Ultimately, the presence of measurement error affects the confidence with which a classification decision may be made for a given particle or cell.

Currently, flow cytometry data does not comprise any information about measurement uncertainty at the event-specific or parameter-specific or population-specific levels. Practitioners are required to manually measure, calculate, or estimate sources of measurement uncertainty.

### SUMMARY

Thus, the inventors have realized that there is a need for automatically reporting measurement uncertainty and classification uncertainty of flow cytometry data through, for example, quantitative quality scores. In particular, there is a need for improved ability to differentiate true biological variability from measurement error. The present disclosure addresses this need. The present disclosure addresses limitations of existing techniques by associating event-specific measurement uncertainties with the event data, for example. A method for estimating event-specific measurement uncertainty is provided. Such improvements of existing techniques may, among other things, improve sort purity and yield.

An aspect of the present disclosure includes a method for identifying measurement uncertainty associated with light detected from a sample. The method according to the present disclosure includes introducing a sample into a flow cytometer, flowing the introduced sample in a flow stream, irradiating the sample in the flow stream with a light source, detecting light from particles in the sample flowing in the flow stream and, identifying measurement uncertainty associated with the detected light. Measurement uncertainty can be identified corresponding to individual particles in the sample. Measurement uncertainty can be identified for individual parameters of detected light for particles in the sample. The present disclosure can further comprise generating a quality score for each particle based on the measurement uncertainty for each particle. A system, integrated circuit device (e.g., a field programmable gate array) and non-transitory computer readable storage medium for practicing the subject method are also provided.

### BRIEF DESCRIPTION OF THE FIGURES

The disclosure may be best understood from the following detailed description when read in conjunction with the accompanying drawings. Included in the drawings are the following figures:

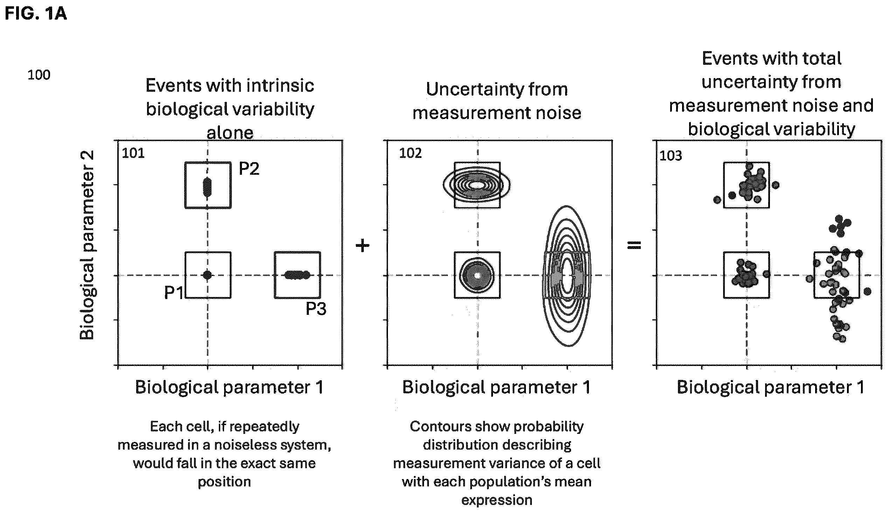

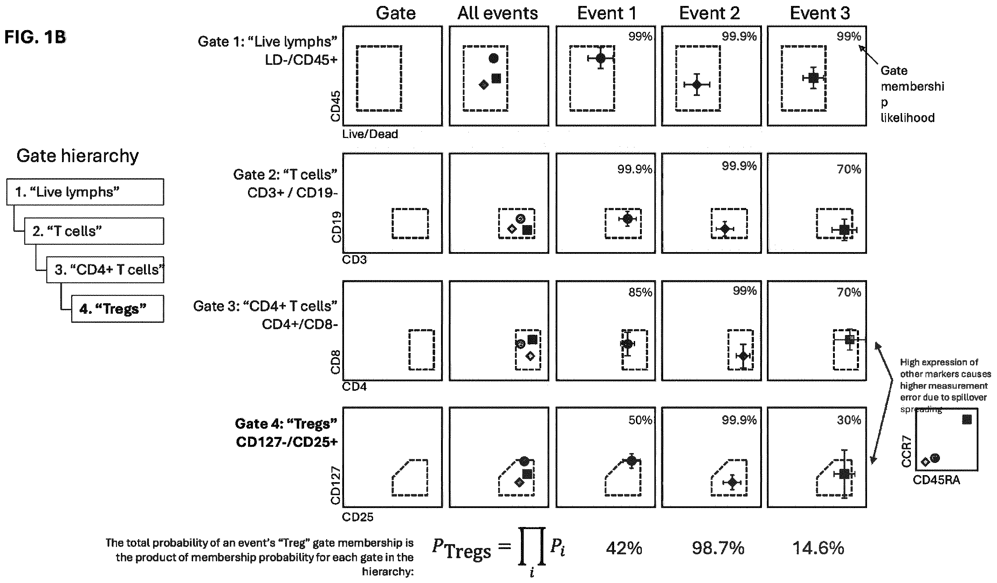

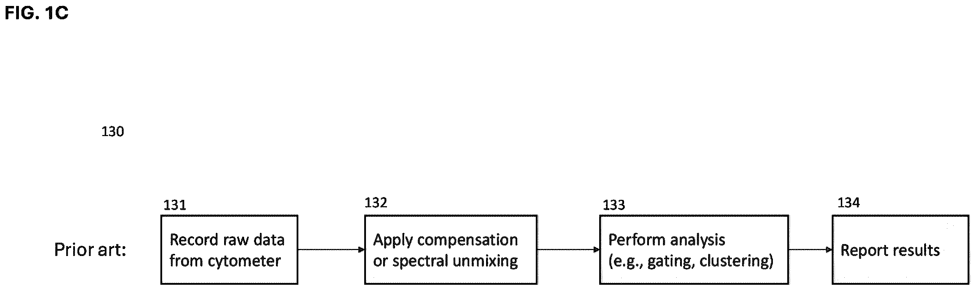

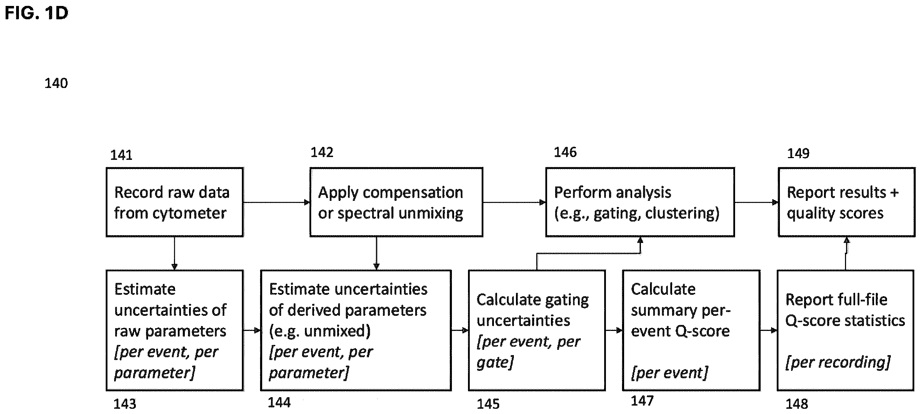

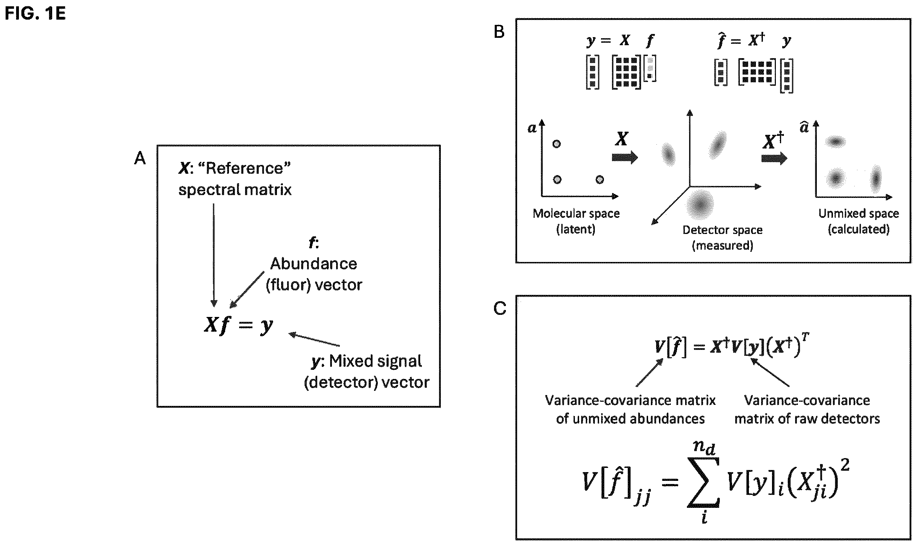

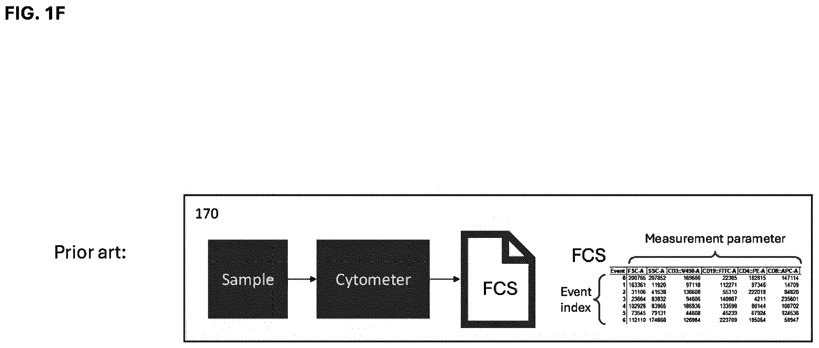

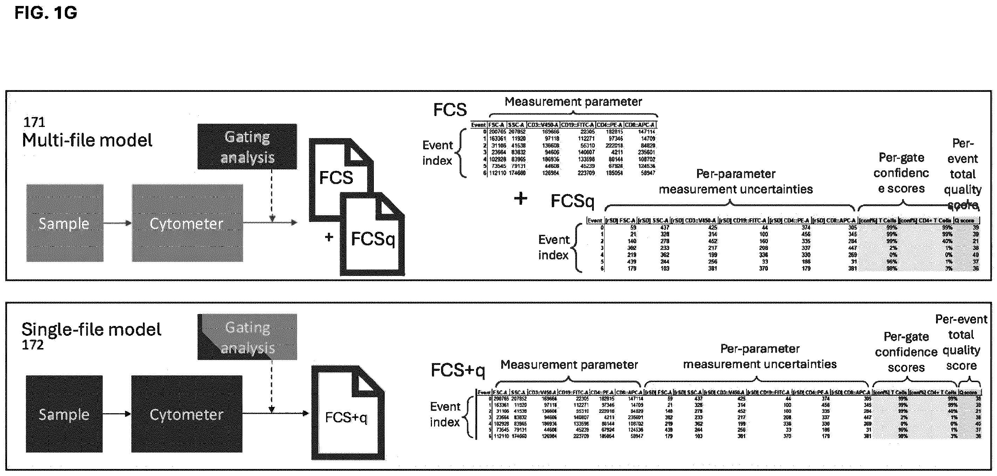

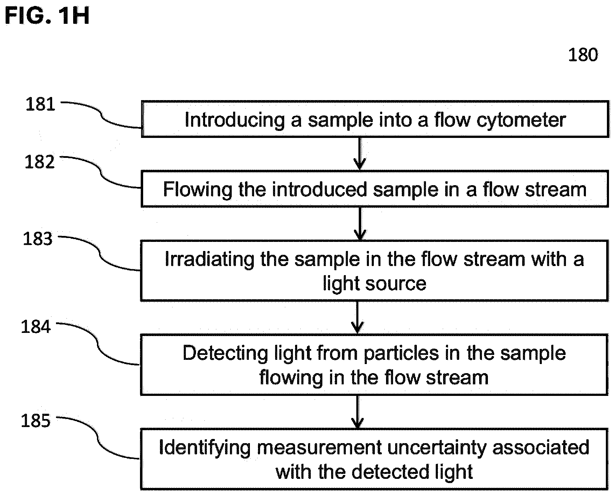

**FIG. 1A** shows exemplary results 100 of flow cytometric measurements of events with associated biological and measurement uncertainties. **FIG. 1B** shows exemplary hierarchical gates applied to events of a sample. **FIG. 1C** shows techniques for addressing measurement uncertainty according to the prior art. **FIG. 1D** shows a technique for identifying measurement uncertainty on per-parameter, per-event per-gate or per-sample or per-recording basis, according to the present disclosure. **FIG. 1E** shows an exemplary processes for estimating measurement uncertainty, according to a "GLS unmixing approach." **FIG. 1F** shows measurement data storage according to prior art. **FIG. 1G** shows techniques for recording measurement data along with measurement uncertainty data according to the present disclosure. **FIG. 1H** shows a flow diagram for identifying measurement uncertainty and classification uncertainty of flow cytometry data according to the present disclosure.
**FIG. 2** presents a flow cytometric system according to the present disclosure.
**FIG. 3** depicts an image-enabled particle sorter according to the present disclosure.
**FIG. 4** depicts a functional block diagram of a particle analysis system according to the present disclosure.
**FIG. 5** depicts a depicts a functional block diagram for one example of a control system according to the present disclosure.
**FIG. 6A-6B** depict schematic drawings of a particle sorter system according to the present disclosure.
**FIG. 7** depicts aspects of a computer-controlled system according to the present disclosure.

### DETAILED DESCRIPTION

Aspects of the present disclosure include methods for identifying measurement uncertainty associated with light detected from a sample. Methods according to the present disclosure include introducing a sample into a flow cytometer, flowing the introduced sample in a flow stream, irradiating the sample in the flow stream with a light source, detecting light from particles in the sample flowing in the flow stream and, identifying measurement uncertainty associated with the detected light. In the present disclosure, measurement uncertainty is identified corresponding to individual particles in the sample. In the present disclosure, measurement uncertainty is identified for individual parameters of detected light for particles in the sample. Methods according to the present disclosure further comprise generating a quality score for each particle based on the measurement uncertainty for each particle. Systems, integrated circuit devices (e.g., a field programmable gate array) and non-transitory computer readable storage mediums for practicing the subject methods are also provided.

Before the present disclosure is described in greater detail, it is to be understood that this disclosure is not limited to the present disclosure described, as such may, of course, vary. It is also to be understood that the terminology used herein is for the purpose of describing the present disclosure only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, representative illustrative methods and materials are now described.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present disclosure is not entitled to antedate such publication by virtue of prior disclosure. Further, the dates of publication provided may be different from the actual publication dates which may need to be independently confirmed.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As summarized above, the present disclosure provides a method for identifying measurement uncertainty associated with light detected from a sample. The method can include identifying measurement uncertainty associated with light detected from a sample, including identifying measurement uncertainty corresponding to individual particles in the sample as well as identifying measurement uncertainty for individual parameters of detected light for particles in the sample as well as generating a quality score for each particle based on the measurement uncertainty for each particle. Next, systems, integrated circuit devices and non-transitory computer readable storage mediums, in each case programmed to practice the subject methods, are described.

### Background:

As discussed herein, flow cytometry is used to measure features of single particles (also referred to as microparticles) or cells based on optical signals (i.e., detected light). A frequent use case of flow cytometry is identifying different cell subpopulations (or cell populations) or particle types based on differences in measured optical signals, such as the quantity of emitted fluorescence from fluorochrome-labeled antibodies in one or more spectral detection bands, or the quantity of elastically-scattered light measured at different angles (e.g., forward scatter and side scatter). To effectively identify and separate populations of cells or particles based on these measured signals, practitioners must be able to determine whether a measured difference in signal between two particles is due to a true intrinsic difference between those particles (e.g., a difference in expression of a biological marker, or a difference in cell size or morphology), or whether the measured difference in signal is due to random measurement error (also called measurement noise, measurement uncertainty, or measurement variability). **FIG. 1A** depicts exemplary results 100 of flow cytometric measurements of events, illustrating how overall variation in such measurements 103 is comprised of intrinsic biological variability alone 101 combined with uncertainty from measurement noise 102. **FIG. 1B** shows exemplary hierarchical gates applied to events of a sample. Ultimately, the presence of measurement error affects the confidence with which a classification decision (i.e., gating) may be made for a given microparticle, as illustrated in **FIG. 1B**.

Measurement error is unavoidable in flow cytometry and can arise from multiple sources including, for example, electronic noise (e.g., Johnson-Nyquist noise), digitization error, photonic shot noise, or random variations in optical excitation and collection efficiency arising from random fluctuations in illumination intensity, fluidics, optical alignment, photodetector gain, and other hardware components over the course of an experimental measurement. The magnitude of error arising from each of these sources depends on the specific instrument, sample, and measurement conditions (such as, for example, photodetector gain, flow rate, etc.) used for a given sample. The total uncertainty in a flow cytometry sample (i.e., total uncertainty related to measurements of detected light corresponding to events in a flow cytometry sample) arises from a combination of the above-mentioned measurement uncertainty as well as intrinsic variability in the sample itself, such as, for example, randomly varying degrees of expression of a biological molecule on a single biological cell population of interest, or randomly varying degrees of fluorescence intensity in a synthetic fluorescent bead arising from uncontrollable variation in the manufacturing process.

A significant source of measurement uncertainty in multicolor flow cytometry experiments is so-called "spillover-spreading error." *See* Nguyen R, Perfetto S, Mahnke YD, Chattopadhyay P, Roederer M. Quantifying spillover spreading for comparing instrument performance and aiding in multicolor panel design. Cytometry A. 2013 Mar;83(3):306-15. doi: 10.1002/cyto.a.22251. Epub 2013 Feb 6. PMID: 23389989; PMCID: PMC3678531. This manifests as increased variance in spectrally unmixed or compensated measurement parameters - typically the parameters of interest in biological analysis - as a result of photonic shot noise originating from fluorescence emission from one or more fluorophores that are co-expressed with another fluorophore of interest.

The ability to differentiate true biological variability from measurement error is a significant limiting factor for sensitivity and resolving power in flow cytometry assays. The present invention address such limitations by providing novel and inventive techniques for distinguishing and accounting for measurement error or measurement uncertainty, including in the context of flow cytometric experiments.

In certain existing techniques, measurement error in flow cytometry may quantified in one of two ways: (i) first, by using controlled reference samples and protocols to measure different sources of measurement noise *a priori;* or (ii) second, by applying statistical analyses to measured data *a posteriori.*

In the first uncertainty measurement approach according to existing techniques (item (i) above), the measurement error of a given instrument is measured using specific types of controlled reference samples. For example, calibration beads with low intrinsic variability and well-characterized intensities may be used to measure the robust standard deviation (rSD) and robust coefficient of variation (rCV) of a given optical measurement parameter. More complex protocols can enable the measurement of specific noise sources under controlled conditions. For example, a technique that may be employed involves the use of electronic event triggering in the absence of particles to measure signal-independent background noise ("B") under varying system conditions, such as the presence or absence of constant optical noise arising from excitation light sources. In another example, particle-free signal sources such as light-emitting diodes (LEDs) may be used to characterize measurement error arising exclusively from constant background and from photon counting error, in the absence of particle-dependent error sources such as intrinsic intensity variation and fluidic/optical fluctuations. Finally, in another example, representative biological samples, such as single-stain controls or fluorescence-minus-one (FMO) controls, can be used to infer the degree of total uncertainty that would be present in a fully-stained sample of interest in a multicolor flow cytometry panel. An advantage or benefit of this first uncertainty measurement approach (i.e., techniques under item (i) above) is that it allows measurement of specific sources of measurement variance isolated from biological and sample-dependent sources of variation. However, a disadvantage or downside of this first measurement approach (i.e., techniques under item (i) above) is that measurement uncertainty is not measured directly in the sample of interest under that sample's measurement conditions, and, further, measurement uncertainty cannot be associated with specific single cells or particles in a given dataset. Moreover, to apply statistical insights from these separate uncertainty measurements to a given sample of interest, the measurements must be manually associated with each other, and some empirical corrections must be made to account for differences in measurement conditions between the calibration sample and the sample of interest.

In the second uncertainty measurement approach according to existing techniques (item (ii) above), statistical techniques are applied to data from a sample of interest after such data has been acquired. A typical workflow would be to identify a subpopulation of interest via gating on some set of measurement parameters, and then apply appropriate statistical metrics such as robust standard deviation (rSD) or robust coefficient of variation (rCV) to the events in that subpopulation. Additional metrics such as stain index or separation index may be used to define the statistical degree of separation between two subpopulations with respect to a given measurement parameter. Statistical confidence may be gained by performing technical replicates, e.g., measuring the same sample multiple times. An advantage of this second uncertainty measurement approach (item (ii) above) is that it describes uncertainty for the specific sample and population of interest, under the exact measurement conditions used. An important disadvantage of this approach (item (ii) above), however, is that it cannot be used to discriminate between technical measurement uncertainty arising from the measurement process and biological variability arising from the intrinsic biological properties of the sample itself. Such disadvantage or limitation prevents practitioners from differentiating between measurement noise and biologically meaningful variation.

**FIG. 1C** illustrates existing a technique for addressing measurement uncertainty according to the prior art. Flow diagram 130, starts at step 131 with recording raw data from a flow cytometer. Upon completing step 131, flow diagram 130 proceeds to step 132. At step 132, spectral unmixing is performed on data acquired by the flow cytometer at step 131. Compensation or spectral unmixing may be performed using any convenient compensation or spectral unmixing technique. Further details regarding spectral unmixing are provided in International Application No. PCT/US2021/026616, published as International Publication No. WO 2021/221884 A1, as well as International Application No. PCT/US2021/046741, published as International Publication No. WO 2022/076088 A1. Upon completing step 132, flow diagram 130 proceeds to step 133. At step 133, data analysis is performed of flow cytometric data acquired at step 131. Such analysis may include, for example, applying one or more gating or clustering algorithms. Upon completing step 133, flow diagram 130 proceeds to step 134. At step 134, results, i.e., results of processing the flow cytometric data in steps 132 and 133, are reported. Unlike the present invention, flow diagram 130 is not able to identify and report resulting metrics of measurement uncertainty on a per-parameter, per-particle, per-population, and/or per-sample basis.

### The present invention:

The present invention employs an alternative approach, in which the measurement uncertainty of some or all measurement parameters for each particle in a flow cytometry sample are directly estimated, measured, or predicted, and the resulting measurement uncertainty metrics are associated with the flow cytometry sample data. The resulting metric(s) of measurement uncertainty may be identified or reported or recorded on a per-parameter, per-particle, per-population, and/or per-sample basis, or any combination thereof. **FIG. 1D** illustrates a technique for identifying measurement uncertainty on per-parameter, per-event per-gate or per-sample or per-recording basis, according to the present disclosure. Flow diagram 140 starts at step 141 where raw flow cytometric data is recorded. Subsequent processing occurs in subsequent steps of flow diagram 140, where measurement data is addressed in steps 141, 142, 146 and 149, and measurement uncertainty associated with such measurement data is addressed at steps 143, 144, 145, 147 and 148. Such raw cytometric data is further processed at steps 143 and step 142. At step 142, compensation or spectral unmixing may be applied to the raw cytometric data. Such compensation or spectral unmixing results in computing or estimating derived parameters. At step 143, measurement uncertainty is estimated on a per-parameter or per-event basis based on the raw parameter data acquired at step 141. At step 144, measurement uncertainty is estimated based on the derived parameters on a per-parameter or per-event basis. At step 145, gating uncertainties are estimating on a per-event or per-parameter basis. At step 147, a summary of measurement uncertainty is calculated, in this case comprising a per-event Q-score, as discussed herein. At step 147, such results are obtained on a per-recording or per-sample basis. At step 146, analysis, such as applying a gating or clustering algorithm is performed, on the flow cytometric data, in this case on events using the derived parameters (i.e., unmixed data). At step 148, a full file of Q-score statistics are reported, e.g., recorded or displayed. At step 149, full results of analyzed flow cytometric data, including quality scores, such as, for example, Q-scores, are reported, e.g., recorded or displayed. The associated measurement uncertainty data, such as, for example, that calculating in steps 144, 145 and 147 of flow diagram 140, may be used to provide more precise data analysis, to increase statistical confidence in analysis results, to achieve higher-fidelity unsupervised clustering, and to report overall measurement quality and assay performance, among other things.

The present disclosure may be applied to single-cell analysis modalities beyond flow cytometry. For example, the present disclosure may be applied in the context of sequencing-based single-cell proteomics such as CITE-Seq (BD AbSeq).

### Conceptual similarities in the context of next-generation sequencing:

The present invention is similar conceptually to the ubiquitous use of Phred quality scores ("Q scores") in DNA sequencing. A Q score in the context of DNA sequencing is defined as the negative logarithm of the probability that a given base-call is incorrect, reported in logarithmic decibel units (e.g., a Q score of 30 indicates a 1 in 1,000 probability of a base-call error, and a Q score of 40 indicates a 1 in 10,000 probability, etc.). For every base call in every sequencing read in a DNA sequencing experiment, an associated Q score records the probability of the base-call being correct. The sequence data (base calls) and Q score data are then associated with each other and saved, most commonly through the widely used FASTQ data format which combines sequencing data and associated Q scores in a single data file.

In the context of flow cytometry, as in the present invention, a single flow cytometry measurement event (e.g., measurement of a single particle) is analogous to a single sequencing read; a single measurement parameter associated with a measurement event is analogous to a single base-call; a per-parameter, per-event flow cytometry uncertainty score is analogous to a Q score; and a modified FCS or new "FCSQ" format is analogous to the FASTQ format. Several important properties of Q scores are present in the present invention, including, for example, the use of compressed Q score representations to save storage space, and the use of aggregate Q score metrics to indicate the overall read quality of a given sequence (flow cytometry event) or sequencing dataset (flow cytometry sample data).

Some important differences between sequencing Q scores and the present invention include, for example, that sequencing data have a discrete-valued domain (A, C, G, or T) compared to flow cytometry data's continuous value domains (float or integer numbers), and the fact that sequencing errors are described in terms of binary classification error (the base call is either correct or incorrect) while, flow cytometry measurement error for a given parameter can be defined continuously (the measured parameter has some numerical degree of uncertainty described in terms of standard deviation or standard error of the mean, for example). Flow cytometry gating classification is also a binary classification problem (an event belongs within a gated population, or does not, depending on whether corresponding event data falls within the ranges associated with the gated population or does not) and in that sense would exhibit similarly to a sequencing Q score. The term "binary classification" can refer to dividing event data (e.g., flow cytometric events) into two distinct populations (i.e., determining whether an event is classified as belonging to a population or not belonging to such population). In some cases, binary classification of flow cytometric data is performed using classification trees. In some cases, binary classification of flow cytometric data is performed using hierarchical gates. As described above, in the present disclosure, binary classification in the context of flow cytometry is a form of dichotomization in which a continuous function is transformed into a binary variable; continuous values can be made binary by defining a cutoff value; such cutoff value is then used to classify whether the corresponding event is positive or negative based on whether the event data value is higher or lower than the cutoff.

### Uncertainty estimation methods:

There are many methods by which measurement uncertainty metrics used in connection with the present disclosure may be estimated for a flow cytometry dataset. Any convenient technique by which measurement uncertainty may be estimated may be employed. Examples employed are described herein. However, the present invention is not limited to such metrics, and any metric that quantifies measurement uncertainty may be employed. Measurement uncertainty may be calculated from a semi-empirical noise model that combines instrument calibration data, a physical noise model, and the measurement values corresponding to a given event. **FIG. 1E****,** subpanels A, B and C, illustrates an exemplary processes for estimating measurement uncertainty, according to a "GLS unmixing approach." Further details regarding such exemplary technique may be found in U.S. Application No. 18/986,295, which claims priority to U.S. Provisional Application No. 63/622,370.

Measurement uncertainty can be estimated post-hoc on a per-event basis using a statistical algorithm configured to measure properties of data distributions within the sample. A combination of *a priori* noise modeling and *a posteriori* distribution fitting may be employed, using approaches such as Bayesian inference.

### Uncertainty score metrics:

The specific numerical metric used to report uncertainty may comprise any metric of measurement uncertainty and such may vary. Further, the type of metric may vary by parameter. That is, for a given event, in which a plurality of parameter measurements are associated, each parameter may have a different measurement uncertainty metric applied to it. Uncertainty scores may also be reported for binary classification results arising from analysis, e.g., gating, population membership, etc. (Gating in the context of flow cytometry refers to a process used to isolate specific event populations from a larger sample based on characteristics like size, granularity, and fluorescence, etc. Population membership or population gating refers to a process of classification of events into populations or subpopulations based on specific characteristics, such as size, morphology, and protein expression, etc.) These may be reported as an uncertainty score (i.e., in which a higher value indicates higher uncertainty) or as a quality score (i.e., in which a higher value indicates higher confidence). Uncertainty scores for an event of other binary classification results, for example, may be derived from other per-parameter uncertainty scores. Example metrics of interest include, for example: direct quantification (e.g., standard deviation); a confidence interval; a likelihood that an event's measured value is within some percent of its true value; or residuals describing the goodness-of-fit of unmixed data. A confidence interval can be a range of values that is likely to contain the value of an unknown population parameter. Such intervals represent a plausible range for the parameter given the characteristics of the sample. Confidence intervals are derived from sample statistics and are calculated using a specified confidence level. That is, estimates of statistical values based on samples of populations involve uncertainty, and a confidence interval specifies a range of values in which such estimated statistic is expected to fall a certain percentage of the time an experiment is run or a population is re-sampled. The confidence level is the percentage of times it is expected to reproduce an estimate between the upper and lower bounds of the confidence interval. For example, for a confidence interval with a 95% confidence level, it can be expected that 95 out of 100 times an estimate will fall between the upper and lower values specified by the confidence interval.

### Gating quality scores:

The present invention may comprise calculating a gating quality score. A quality score may be calculated that estimates the likelihood of an event's membership in a given gate. This likelihood may be defined for each gate in a gating hierarchy, or may be defined hierarchically where the likelihood of membership in all of a gate's parent gates is taken into account. **FIG. 1B** illustrates hierarchical gates and associated likelihoods of membership for exemplary hierarchical gates.

### Applications:

Uncertainty scores as calculated by the present invention can find use in a number of different contexts, including, but not limited to: assessment of statistical significance or confidence intervals for event classification; use in variance-stabilizing transforms for data visualization and analysis; use in data preprocessing to minimize intra-cluster variance in supervised or unsupervised clustering techniques; use in data standardization to normalize measurement uncertainty across datasets measured on different instruments or under different conditions; and use in probabilistic analysis for cell classification and sorting ("fuzzy logic").

### Format and data storage:

Uncertainty scores or measurements or results could be stored and associated with measurement data (e.g., raw flow cytometric data or compensated or unmixed cytometric data) in any convenient way, and such may vary. **FIG. 1F** illustrates measurement data storage according to existing techniques, in which measurement data is ultimately stored 170 in an FCS file format, but no per-parameter or per-event or per-gate or per-sample or other measure of measurement uncertainty is estimated or calculated or recorded.

**FIG. 1G** shows how the present invention may record measurement data along with measurement uncertainty data. Multi-file model 171 according to an the present disclosure comprises a single shared data record: uncertainty scores are added as additional event-specific FCS parameters (e.g., for measurement parameter "FITC-A", an additional measurement parameter "FITC-A-Uncertainty" would also be saved). This multi-file approach 171 is a simple solution and exhibits certain similarities to the sequencing FASTQ implementation described herein. A disadvantage or downside to the multi-file model approach 171 would be increased file size and data storage requirements. Only FCS3.2+ formats would yield the storage-space benefits of a compressed uncertainty score (e.g., 1 byte instead of 4 bytes) since earlier FCS formats store all values as 32-bit floats.

The present disclosure can utilize a split data record approach 172: a separate data file (for example, named .FCSQ) comprising uncertainty scores would be generated alongside a typical flow data file (.FCS) (i.e., according to existing techniques). The split data record approach may allow for uncertainty scores to be provided for all or only some of the parameters in the .FCS file.

Aggregate uncertainty scores at the population or recording level could be stored in the .FCS file header as additional keywords.

To support reproducibility and traceability, all metadata necessary for generating the uncertainty scores for a given recording (including instrument calibration parameters and noise model information) could be saved in the .FCS file header.

### METHODS

Aspects of the present disclosure include methods for identifying measurement uncertainty associated with light detected from a sample. Methods according to the present disclosure include introducing a sample into a flow cytometer, flowing the introduced sample in a flow stream, irradiating the sample in the flow stream with a light source, detecting light from particles in the sample flowing in the flow stream and, identifying measurement uncertainty associated with the detected light. In the present disclosure, measurement uncertainty is identified corresponding to individual particles in the sample. In the present disclosure, measurement uncertainty is identified for individual parameters of detected light for particles in the sample. Methods according to the present disclosure further comprise generating a quality score for each particle based on the measurement uncertainty for each particle.

Detecting light from particles may comprise measuring a plurality of parameters of the detected light for particles in the sample. The present disclosure can further comprise: generating event data based on the detected light, wherein event data comprises parameter measurements for particles in the sample; spectrally resolving the light detected from particles:spectrally resolving the light detected from particles may produce a plurality of derived parameters. The method of the disclosure may further comprise: generating a plurality of derived parameters for each particle by spectrally resolving the light detected from each particle. The derived parameters may comprise unmixed detected light. Measurement uncertainty can be identified for derived parameters.

The present disclosure may further comprise: identifying a first subset of particles in the sample by applying a first gate to the detected light. Measurement uncertainty can correspond to identifying the first subset of particles. The method can further comprise: identifying a plurality of subsets of particles in the sample by applying a corresponding plurality of gates to the detected light. Measurement uncertainty can correspond to identifying each of the plurality of subsets of particles. Measurement uncertainty can correspond to gate-level uncertainty.

The present disclosure may further comprise: generating a quality score (Q score) for each particle based on the measurement uncertainty for each particle. The present disclosure may further comprise: recording each event and associated measurement uncertainty for each event. The present disclosure may further comprise: recording measurements for each particle and associated measurement uncertainty for each particle. The present disclosure may further comprise: recording measurements for a plurality of parameters for each particle and associated measurement uncertainty for each parameter of each particle. The present disclosure may further comprise: recording measurement uncertainty, wherein the measurement uncertainty corresponds to measurement uncertainty for one or more of: each recorded parameter; each recorded event; each gate or other classification; or for the sample.

The measurement uncertainty for different parameters measured for particles in the sample may comprise a different metric. In the present disclosure, the measurement uncertainty may correspond to a binary classification event. The binary classification event may correspond to one or more of: a gating determination or a population membership classification decision. The measurement uncertainty may comprise a metric that quantifies measurement uncertainty. The present disclosure may further comprise: generating an uncertainty score based on the measurement uncertainty. Measurement uncertainty may be reflected in an uncertainty score, wherein higher value uncertainty scores indicates greater measurement uncertainty. The present disclosure may further comprise : generating a quality score based on the measurement uncertainty. In embodiments, the measurement uncertainty is reflected in a quality score, wherein higher value quality score indicates higher confidence.

The measurement uncertainty may comprise one or more of: direct quantification, optionally comprising a standard deviation; a confidence interval; a likelihood that a measured value is within a specified percent of a true value; or a residual related to a goodness-of-fit of unmixed data. The measurement uncertainty may relate to a binary classification, wherein the binary classification optionally comprises one or more of gating or population membership classifications.

The present disclosure may further comprise : calculating a quality score based on the measurement uncertainty, wherein the quality score reflects a likelihood of membership in a gate. The likelihood of membership in the gate may be calculated for each gate in a gate hierarchy. The likelihood of membership in the gate may be calculated taking into account each hierarchical parent gate of the gate. Identifying measurement uncertainty associated with the detected light may comprise: calculating measurement uncertainty based on a semi-empirical noise model, wherein the semi-empirical noise model comprises one or more of: instrument calibration data, a physical noise model, or measurement values. Identifying measurement uncertainty associated with the detected light may comprise: estimating measurement uncertainty on a per event basis based on a statistical algorithm, wherein the statistical algorithm comprises measuring properties of data distributions within the sample.

Identifying measurement uncertainty associated with the detected light may comprise: estimating measurement uncertainty comprises applying a combination of noise modeling and distribution fitting. present Identifying measurement uncertainty associated with the detected light may comprise: estimating measurement uncertainty comprises applying Bayesian inference.

The present disclosure may further comprise : reporting measurement uncertainty for each particle of a subset of particles of the sample. The present disclosure may further comprise : reporting measurement uncertainty for a plurality of parameters for each particle of a subset of particles of the sample. The present disclosure may further comprise: reporting measurement uncertainty associated with membership in a subpopulation defined by a gate for each particle in a subset of particles.

Measurement uncertainty may comprise random measurement error. Measurement uncertainty may comprise spillover-spreading error. Measurement uncertainty may reflect variations other than true intrinsic differences between particles of the sample. Measurement uncertainty may reflect confidence in a classification decision regarding particles of the sample. Measurement uncertainty may reflect an aggregate measurement uncertainty related to a plurality of parameter measurements of light detected from particles. Measurement uncertainty may reflect an aggregate measurement uncertainty related to measurements of light detected from a plurality of particles. Measurement uncertainty may reflect an aggregate measurement uncertainty related to the sample.

Identifying measurement uncertainty associated with the detected light may comprise one or more of: estimating measurement uncertainty associated with the detected light, measuring measurement uncertainty associated with the detected light, or predicting measurement uncertainty associated with the detected light.

The present disclosure may further comprise : classifying particles based on detected light; and calculating a statistical significance for classification of particles based at least in part on the measurement uncertainty. The present disclosure may further comprise : classifying particles based on detected light; and calculating a confidence interval for classification of particles based at least in part on the measurement uncertainty. The present disclosure may further comprise : applying a variance-stabilizing transform to data comprising measurements of light detected from particles in the sample based at least in part on the measurement uncertainty; and visualizing aspects of the transformed data. The present disclosure may further comprise : preprocessing data comprising measurements of light detected from particles in the sample to minimize intra-cluster variance based at least in part on the measurement uncertainty; and applying a clustering algorithm to the preprocessed data, wherein the clustering algorithm optionally comprises one or more of a supervised clustering algorithm and an unsupervised clustering algorithm. The present disclosure may further comprise: standardizing data comprising measurements of light detected from particles in the sample to normalize measurement uncertainty across different datasets based at least in part on the measurement uncertainty, wherein the different data sets optionally comprise one or more of datasets collected on different instruments or datasets collected under different conditions. The present disclosure may further comprise : using the measurement uncertainty for probabilistic analysis of particle classification or sorting, wherein the probabilistic classification optionally comprises applying a fuzzy logic technique.

The method can be a method of distinguishing true variability in the particles of the sample from measurement error. In other embodiments, the method is a method of distinguishing true biological variability from measurement error. The method can be a method of improving sensitivity of flow cytometry assays. The method can be a method of improving resolving power of flow cytometry assays. The method can be a method for assessing a quality of particle analysis results by associating event-specific estimates of measurement uncertainty with flow cytometry measurements.

A quantitative metric of measurement uncertainty may be associated with flow cytometry measurement. The present disclosure may further comprise: using measurement uncertainty in connection with data analysis or sorting. The present disclosure may further comprise: identifying a gate membership confidence score for each event and each gate, wherein the gate membership confidence score comprises a likelihood that a true biological expression level for a given event falls within a given gate.

The method can be a method for calculating gate membership confidence scores. The present disclosure may further comprise further comprise: using gate membership confidence scores based on measurement uncertainty in particle classification and sorting, wherein particle classification and sorting comprises probabilistic sorting with configurable likelihood thresholds to maximize purity and/or yield.

Light can be detected with a light detection system. Light can be detected by the light detection system in a plurality of photodetector channels. The light detection system may comprise a plurality of photodetectors.

**FIG. 1H** depicts a flow diagram 180 for identifying measurement uncertainty and classification uncertainty of flow cytometry data according to the present disclosure. As discussed in greater detail herein, at step 181, a sample is introduced into a flow cytometer; at step 182, the sample is flowed in a flow stream; at step 183, the sample in the flow stream is irradiated with a light source; at step 184, light is detected from particles in the sample flowing in the flow stream; at step 185, measurement uncertainty associated with the detected light is identified. Further details regarding each of steps 181, 182, 183, 184 and 185 are described herein.

The sample analyzed in the instant methods can be a biological sample. The term "biological sample" is used in its conventional sense to refer to a whole organism, plant, fungi or a subset of animal tissues, cells or component parts which may in certain instances be found in blood, mucus, lymphatic fluid, synovial fluid, cerebrospinal fluid, saliva, bronchoalveolar lavage, amniotic fluid, amniotic cord blood, urine, vaginal fluid and semen. As such, a "biological sample" refers to both the native organism or a subset of its tissues as well as to a homogenate, lysate or extract prepared from the organism or a subset of its tissues, including but not limited to, for example, plasma, serum, spinal fluid, lymph fluid, sections of the skin, respiratory, gastrointestinal, cardiovascular, and genitourinary tracts, tears, saliva, milk, blood cells, tumors, organs. Biological samples may be any type of organismic tissue, including both healthy and diseased tissue (e.g., cancerous, malignant, necrotic, etc.). The biological sample can be a liquid sample, such as blood or derivative thereof, e.g., plasma, tears, urine, semen, etc., where in some instances the sample is a blood sample, including whole blood, such as blood obtained from venipuncture or fingerstick (where the blood may or may not be combined with any reagents prior to assay, such as preservatives, anticoagulants, etc.).

The source of the sample can be a "mammal" or "mammalian", where these terms are used broadly to describe organisms which are within the class Mammalia, including the orders carnivore (e.g., dogs and cats), Rodentia (e.g., mice, guinea pigs, and rats), and primates (e.g., humans, chimpanzees, and monkeys).The subjects can be humans. The method may be applied to samples obtained from human subjects of both genders and at any stage of development (i.e., neonates, infant, juvenile, adolescent, adult), where the human subject is a juvenile, adolescent or adult. While the present disclosure may be applied to samples from a human subject, it is to be understood that the methods may also be carried-out on samples from other animal subjects (that is, in "non-human subjects") such as, but not limited to, birds, mice, rats, dogs, cats, livestock and horses.

Cells of interest may be targeted for characterized according to a variety of parameters, such as a phenotypic characteristic identified via the attachment of a particular fluorescent label to cells of interest. The disclosure may be configured to deflect analyzed droplets that are determined to include a target cell. A variety of cells may be characterized using the subject methods. Target cells of interest include, but are not limited to, stem cells, T cells, dendritic cells, B Cells, granulocytes, leukemia cells, lymphoma cells, virus cells (e.g., HIV cells), NK cells, macrophages, monocytes, fibroblasts, epithelial cells, endothelial cells, and erythroid cells. Target cells of interest include cells that have a convenient cell surface marker or antigen that may be captured or labelled by a convenient affinity agent or conjugate thereof. For example, the target cell may include a cell surface antigen such as CD11b, CD123, CD14, CD15, CD16, CD19, CD193, CD2, CD25, CD27, CD3, CD335, CD36, CD4, CD43, CD45RO, CD56, CD61, CD7, CD8, CD34, CD1c, CD23, CD304, CD235a, T cell receptor alpha/beta, T cell receptor gamma/delta, CD253, CD95, CD20, CD105, CD117, CD120b, Notch4, Lgr5 (N-Terminal), SSEA-3, TRA-1-60 Antigen, Disialoganglioside GD2 and CD71. The target cell can be selected from HIV containing cell, a Treg cell, an antigen-specific T - cell populations, tumor cells or hematopoietic progenitor cells (CD34+) from whole blood, bone marrow or cord blood.

In practicing the present disclosure an amount of an initial fluidic sample can be injected into the flow cytometer. The amount of sample injected into the particle sorting module may vary, for example, ranging from 0.001 mL to 1000 mL, such as from 0.005 mL to 900 mL, such as from 0.01 mL to 800 mL, such as from 0.05 mL to 700 mL, such as from 0.1 mL to 600 mL, such as from 0.5 mL to 500 mL, such as from 1 mL to 400 mL, such as from 2 mL to 300 mL and including from 5 mL to 100 mL of sample.

The present disclosure may include counting and optionally sorting labeled particles (e.g., target cells) in a sample. In practicing the subject methods, the fluidic sample including the particles is first introduced into a flow nozzle of the system. Upon exit from the flow nozzle, the particles are passed substantially one at a time through the sample interrogation region where each of the particles is irradiated to a source of light and measurements of light scatter parameters and, in some instances, fluorescent emissions as desired (e.g., two or more light scatter parameters and measurements of one or more fluorescent emissions) are separately recorded for each particle. Depending on the properties of the flow stream being interrogated, 0.001 mm or more of the flow stream may be irradiated with light, such as 0.005 mm or more, such as 0.01 mm or more, such as 0.05 mm or more, such as 0.1 mm or more, such as 0.5 mm or more and including 1 mm or more of the flow stream may be irradiated with light.The method may include irradiating a planar cross-section of the flow stream in the sample interrogation region, such as with a laser (as described above). The method may include irradiating a predetermined length of the flow stream in the sample interrogation region, such as corresponding to the irradiation profile of a diffuse laser beam or lamp.

The method may include irradiating the flow stream at or near the flow cell nozzle orifice. For example, irradiating the flow stream at a position about 0.001 mm or more from the nozzle orifice, such as 0.005 mm or more, such as 0.01 mm or more, such as 0.05 mm or more, such as 0.1 mm or more, such as 0.5 mm or more and including 1 mm or more from the nozzle orifice. The method may include irradiating the flow stream immediately adjacent to the flow cell nozzle orifice.

Detectors, such as photomultiplier tubes (PMT), may be used to record light that passes through each particle (in certain cases referred to as forward light scatter), light that is reflected orthogonal to the direction of the flow of the particles through the sensing region (in some cases referred to as orthogonal or side light scatter) and fluorescent light emitted from the particles, if it is labeled with fluorescent marker(s), as the particle passes through the sensing region and is illuminated by the energy source. Each of forward light scatter (FSC), side-scatter (SSC), and fluorescence emissions include a separate parameter for each particle (or each "event"). Thus, for example, two, three or four parameters can be collected (and recorded) from a particle labeled with two different fluorescence markers. The data recorded for each particle is analyzed in real time or stored in a data storage and analysis means, such as a computer, as desired.

The particles may be detected and uniquely identified by exposing the particles to excitation light and measuring the fluorescence of each particle in one or more detection channels, as desired. Fluorescence emitted in detection channels used to identify the particles and binding complexes associated therewith may be measured following excitation with a single light source, or may be measured separately following excitation with distinct light sources. If separate excitation light sources are used to excite the particle labels, the labels may be selected such that all the labels are excitable by each of the excitation light sources used.

The present disclosure may further also include data acquisition, analysis and recording, such as with a computer, wherein multiple data channels record data from each detector for the light scatter and fluorescence emitted by each particle as it passes through the sample interrogation region of the particle sorting module. Analysis may include classifying and counting particles such that each particle is present as a set of digitized parameter values. The subject systems may be set to trigger on a selected parameter in order to distinguish the particles of interest from background and noise. "Trigger" refers to a preset threshold for detection of a parameter and may be used as a means for detecting passage of a particle through the light source. Detection of an event that exceeds the threshold for the selected parameter triggers acquisition of light scatter and fluorescence data for the particle. Data is not acquired for particles or other components in the medium being assayed which cause a response below the threshold. The trigger parameter may be the detection of forward-scattered light caused by passage of a particle through the light beam. The flow cytometer then detects and collects the light scatter and fluorescence data for the particle.

A particular subpopulation of interest is then further analyzed by "gating" based on the data collected for the entire population. To select an appropriate gate, the data is plotted so as to obtain the best separation of subpopulations possible. This procedure may be performed by plotting forward light scatter (FSC) vs. side (i.e., orthogonal) light scatter (SSC) on a two dimensional dot plot. A subpopulation of particles is then selected (i.e., those cells within the gate) and particles that are not within the gate are excluded. Where desired, the gate may be selected by drawing a line around the desired subpopulation using a cursor on a computer screen. Only those particles within the gate are then further analyzed by plotting the other parameters for these particles, such as fluorescence. Where desired, the above analysis may be configured to yield counts of the particles of interest in the sample.

The present disclosure may further include employing particles in research, laboratory testing, or therapy. The present disclosure may further include obtaining individual cells prepared from a target fluidic or tissue biological sample. For example, obtaining cells from fluidic or tissue samples to be used as a research or diagnostic specimen for diseases such as cancer. Likewise, the present disclosure may further include obtaining cells from fluidic or tissue samples to be used in therapy. A cell therapy protocol is a protocol in which viable cellular material including, e.g., cells and tissues, may be prepared and introduced into a subject as a therapeutic treatment. Conditions that may be treated by the administration of the flow cytometrically sorted sample include, but are not limited to, blood disorders, immune system disorders, organ damage, etc.

A typical cell therapy protocol may include the following steps: sample collection, cell isolation, genetic modification, culture, and expansion in vitro, cell harvesting, sample volume reduction and washing, bio-preservation, storage, and introduction of cells into a subject. The protocol may begin with the collection of viable cells and tissues from source tissues of a subject to produce a sample of cells and/or tissues. The sample may be collected via any suitable procedure that includes, e.g., administering a cell mobilizing agent to a subject, drawing blood from a subject, removing bone marrow from a subject, etc. After collecting the sample, cell enrichment may occur via several methods including, e.g., centrifugation based methods, filter based methods, elutriation, magnetic separation methods, fluorescence-activated cell sorting (FACS), and the like. In some cases, the enriched cells may be genetically modified by any convenient method, e.g., nuclease mediated gene editing. The genetically modified cells can be cultured, activated, and expanded in vitro. In some cases, the cells are preserved, e.g., cryopreserved, and stored for future use where the cells are thawed and then administered to a patient, e.g., the cells may be infused in the patient.

### SYSTEMS FOR IDENTIFYING MEASUREMENT UNCERTAINTY ASSOCIATED WITH LIGHT DETECTED FROM A SAMPLE

As summarized above, present disclosure includes a system for identifying measurement uncertainty associated with light detected from a sample. The system can be configured for practicing the subject methods. Aspects of the disclosure also include flow cytometers. Flow cytometers of interest include a light source configured to irradiate the particles in the flow stream at an interrogation point within a flow cell. Flow cytometers of interest further include a light detection system comprising a plurality of photodetectors.

Flow cells of interest include a cuvette configured to transport particles in a flow stream. As discussed herein, a "flow cell" is described in its conventional sense to refer to a component containing a flow channel for a liquid flow stream for transporting particles in a sheath fluid. Cuvettes of interest have a passage (i.e., flow channel) running therethrough. The flow stream for which the flow channel is configured may include a liquid sample injected from a sample tube. In certain instances, the flow cell includes a light-accessible flow channel. The cuvette may be comprised of, e.g., quartz, glass, clear plastic, and the like. Cuvettes can be formed from silica, such as fused silica. The flow cell can be configured for irradiation with light from a light source at one or more interrogation points. The "interrogation point" discussed herein refers to a region within the flow cell in which the particle is irradiated by light from the light source, e.g., for analysis. The size of the interrogation point may vary as desired. For example, where 0 µm represents the optical axis of light emitted by the light source, the interrogation point may range from -50 µm to 50 µm, such as -25 µm to 40 µm, and including -15 µm to 30 µm. Depending on certain considerations (e.g., the number and arrangement of lasers), multiple irradiation points may exist within the flow cells.

The flow cell may include, or can be configured for use with, a sample injection port configured to provide a sample to the flow cell. The sample injection system can be configured to provide suitable flow of sample to the flow cell inner chamber (i.e., flow channel). Depending on the desired characteristics of the flow stream, the rate of sample conveyed to the flow cell chamber by the sample injection port may be1 µL/min or more, such as 2 µL/min or more, such as 3 µL/min or more, such as 5 µL/min or more, such as 10 µL/min or more, such as 15 µL/min or more, such as 25 µL/min or more, such as 50 µL/min or more and including 100 µL/min or more, where in some instances the rate of sample conveyed to the flow cell chamber by the sample injection port is 1µL/sec or more, such as 2 µL/sec or more, such as 3 µL/sec or more, such as 5 µL/sec or more, such as 10 µL/sec or more, such as 15 µL/sec or more, such as 25 µL/sec or more, such as 50 µL/sec or more and including 100 µL/sec or more.

The sample injection port may be an orifice positioned in a wall of the inner chamber or may be a conduit positioned at the proximal end of the inner chamber. Where the sample injection port is an orifice positioned in a wall of the inner chamber, the sample injection port orifice may be any suitable shape where cross-sectional shapes of interest include, but are not limited to: rectilinear cross-sectional shapes, e.g., squares, rectangles, trapezoids, triangles, hexagons, etc., curvilinear cross-sectional shapes, e.g., circles, ovals, etc., as well as irregular shapes, e.g., a parabolic bottom portion coupled to a planar top portion. The sample injection port may have a circular orifice. The size of the sample injection port orifice may vary depending on shape, in certain instances, having an opening ranging from 0.1 mm to 5.0 mm, e.g., 0.2 to 3.0 mm, e.g., 0.5 mm to 2.5 mm, such as from 0.75 mm to 2.25 mm, such as from 1 mm to 2 mm and including from 1.25 mm to 1.75 mm, for example 1.5 mm.

The sample injection port may be a conduit positioned at a proximal end of the flow cell inner chamber. For example, the sample injection port may be a conduit positioned to have the orifice of the sample injection port in line with the flow cell orifice. Where the sample injection port is a conduit positioned in line with the flow cell orifice, the cross-sectional shape of the sample injection tube may be any suitable shape where cross-sectional shapes of interest include, but are not limited to: rectilinear cross-sectional shapes, e.g., squares, rectangles, trapezoids, triangles, hexagons, etc., curvilinear cross-sectional shapes, e.g., circles, ovals, as well as irregular shapes, e.g., a parabolic bottom portion coupled to a planar top portion. The orifice of the conduit may vary depending on shape, in certain instances, having an opening ranging from 0.1 mm to 5.0 mm, e.g., 0.2 to 3.0 mm, e.g., 0.5 mm to 2.5 mm, such as from 0.75 mm to 2.25 mm, such as from 1 mm to 2 mm and including from 1.25 mm to 1.75 mm, for example 1.5 mm. The shape of the tip of the sample injection port may be the same or different from the cross-section shape of the sample injection tube. For example, the orifice of the sample injection port may include a beveled tip having a bevel angle ranging from 1° to 10°, such as from 2° to 9°, such as from 3° to 8°, such as from 4° to 7° and including a bevel angle of 5°.

The flow cell may also include a sheath fluid injection port configured to provide a sheath fluid to the flow cell. In embodiments, the sheath fluid injection system is configured to provide a flow of sheath fluid to the flow cell inner chamber, for example in conjunction with the sample to produce a laminated flow stream of sheath fluid surrounding the sample flow stream. Depending on the desired characteristics of the flow stream, the rate of sheath fluid conveyed to the flow cell chamber by the may be 25 µL/sec or more, such as 50 µL/sec or more, such as 75 µL/sec or more, such as 100 µL/sec or more, such as 250 µL/sec or more, such as 500 µL/sec or more, such as 750 µL/sec or more, such as 1000 µL/sec or more and including 2500 µL/sec or more.

In The sheath fluid injection port may be an orifice positioned in a wall of the inner chamber. The sheath fluid injection port orifice may be any suitable shape where cross-sectional shapes of interest include, but are not limited to: rectilinear cross-sectional shapes, e.g., squares, rectangles, trapezoids, triangles, hexagons, etc., curvilinear cross-sectional shapes, e.g., circles, ovals, as well as irregular shapes, e.g., a parabolic bottom portion coupled to a planar top portion. The size of the sheath fluid injection port orifice may vary depending on shape, in certain instances, having an opening ranging from 0.1 mm to 5.0 mm, e.g., 0.2 mm to 3.0 mm, e.g., 0.5 mm to 2.5 mm, such as from 0.75 mm to 2.25 mm, such as from 1 mm to 2 mm and including from 1.25 mm to 1.75 mm, for example 1.5 mm.

Flow cytometers of the present disclosure include a light source configured to irradiate the particles in the flow stream at an interrogation point within the flow cell. The number of light sources in the flow cytometers may vary. Flow cytometers may include a single light source. Alternatively, flow cytometers may in some instances include a plurality of light sources. In some such instances, the number of light sources ranges from 2 to 10, such as 2 to 5, and including 2 to 4. Any convenient light source may be employed as the light source described herein. The light source can be a laser. The laser may be any convenient laser, such as a continuous wave laser. For example, the laser may be a diode laser, such as an ultraviolet diode laser, a visible diode laser and a near-infrared diode laser. The laser may be a helium-neon (HeNe) laser. The laser may be a gas laser, such as a helium-neon laser, argon laser, krypton laser, xenon laser, nitrogen laser, CO₂ laser, CO laser, argon-fluorine (ArF) excimer laser, krypton-fluorine (KrF) excimer laser, xenon chlorine (XeCl) excimer laser or xenon-fluorine (XeF) excimer laser or a combination thereof. In other instances, the subject flow cytometers include a dye laser, such as a stilbene, coumarin or rhodamine laser. Lasers of interest may include a metal-vapor laser, such as a helium-cadmium (HeCd) laser, helium-mercury (HeHg) laser, helium-selenium (HeSe) laser, helium-silver (HeAg) laser, strontium laser, neon-copper (NeCu) laser, copper laser or gold laser and combinations thereof. The subject flow cytometers may include a solid-state laser, such as a ruby laser, an Nd:YAG laser, NdCrYAG laser, Er:YAG laser, Nd:YLF laser, Nd:YVO₄ laser, Nd:YCa₄O(BO₃)₃ laser, Nd:YCOB laser, titanium sapphire laser, thulium YAG laser, ytterbium YAG laser, ytterbium₂O₃ laser or cerium doped lasers and combinations thereof.

Laser light sources may also include one or more optical adjustment components.The optical adjustment component may be located between the light source and the flow cell, and may include any device that is capable of changing the spatial width of irradiation or some other characteristic of irradiation from the light source, such as for example, irradiation direction, wavelength, beam width, beam intensity and focal spot. Optical adjustment protocols may include any convenient device which adjusts one or more characteristics of the light source, including but not limited to lenses, mirrors, filters, fiber optics, wavelength separators, pinholes, slits, collimating protocols and combinations thereof. Flow cytometers of interest may include one or more focusing lenses. The focusing lens, in one example, may be a de-magnifying lens. Flow cytometers of interest may include fiber optics.

The light source may be positioned any suitable distance from the flow cell, such as where the light source and the flow cell are separated by 0.005 mm or more, such as 0.01 mm or more, such as 0.05 mm or more, such as 0.1 mm or more, such as 0.5 mm or more, such as 1 mm or more, such as 5 mm or more, such as 10 mm or more, such as 25 mm or more and including at a distance of 100 mm or more. In addition, the light source may be positioned at any suitable angle relative to the flow cell, such as at an angle ranging from 10 degrees to 90 degrees, such as from 15 degrees to 85 degrees, such as from 20 degrees to 80 degrees, such as from 25 degrees to 75 degrees and including from 30 degrees to 60 degrees, for example at a 90 degree angle.

Light sources of interest may include a plurality of lasers configured to provide laser light for discrete irradiation of the flow stream, such as 2 lasers or more, such as 3 lasers or more, such as 4 lasers or more, such as 5 lasers or more, such as 10 lasers or more, and including 15 lasers or more configured to provide laser light for discrete irradiation of the flow stream. Depending on the desired wavelengths of light for irradiating the flow stream, each laser may have a specific wavelength that varies from 200 nm to 1500 nm, such as from 250 nm to 1250 nm, such as from 300 nm to 1000 nm, such as from 350 nm to 900 nm and including from 400 nm to 800 nm.Lasers of interest may include one or more of a 405 nm laser, a 488 nm laser, a 561 nm laser and a 635 nm laser.

The light source may be a light beam generator that is configured to generate two or more beams of frequency shifted light. The light beam generator may include a laser, a radiofrequency generator configured to apply radiofrequency drive signals to an acousto-optic device to generate two or more angularly deflected laser beams. The laser may be a pulsed lasers or continuous wave laser. For example, lasers in light beam generators of interest include those listed above.

The acousto-optic device may be any convenient acousto-optic protocol configured to frequency shift laser light using applied acoustic waves. The acousto-optic device may be an acousto-optic deflector. The acousto-optic device can be configured to generate angularly deflected laser beams from the light from the laser and the applied radiofrequency drive signals. The radiofrequency drive signals may be applied to the acousto-optic device with any suitable radiofrequency drive signal source, such as a direct digital synthesizer (DDS), arbitrary waveform generator (AWG), or electrical pulse generator.

A controller can be configured to apply radiofrequency drive signals to the acousto-optic device to produce the desired number of angularly deflected laser beams in the output laser beam, such as being configured to apply 3 or more radiofrequency drive signals, such as 4 or more radiofrequency drive signals, such as 5 or more radiofrequency drive signals, such as 6 or more radiofrequency drive signals, such as 7 or more radiofrequency drive signals, such as 8 or more radiofrequency drive signals, such as 9 or more radiofrequency drive signals, such as 10 or more radiofrequency drive signals, such as 15 or more radiofrequency drive signals, such as 25 or more radiofrequency drive signals, such as 50 or more radiofrequency drive signals and including being configured to apply 100 or more radiofrequency drive signals.

To produce an intensity profile of the angularly deflected laser beams in the output laser beam, the controller can be configured to apply radiofrequency drive signals having an amplitude that varies such as from about 0.001 V to about 500 V, such as from about 0.005 V to about 400 V, such as from about 0.01 V to about 300 V, such as from about 0.05 V to about 200 V, such as from about 0.1 V to about 100 V, such as from about 0.5 V to about 75 V, such as from about 1 V to 50 V, such as from about 2 V to 40 V, such as from 3 V to about 30 V and including from about 5 V to about 25 V. Each applied radiofrequency drive signal can have , a frequency of from about 0.001 MHz to about 500 MHz, such as from about 0.005 MHz to about 400 MHz, such as from about 0.01 MHz to about 300 MHz, such as from about 0.05 MHz to about 200 MHz, such as from about 0.1 MHz to about 100 MHz, such as from about 0.5 MHz to about 90 MHz, such as from about 1 MHz to about 75 MHz, such as from about 2 MHz to about 70 MHz, such as from about 3 MHz to about 65 MHz, such as from about 4 MHz to about 60 MHz and including from about 5 MHz to about 50 MHz.

The controller may have a processor having memory operably coupled to the processor such that the memory includes instructions stored thereon, which when executed by the processor, cause the processor to produce an output laser beam with angularly deflected laser beams having a desired intensity profile. For example, the memory may include instructions to produce two or more angularly deflected laser beams with the same intensities, such as 3 or more, such as 4 or more, such as 5 or more, such as 10 or more, such as 25 or more, such as 50 or more and including memory may include instructions to produce 100 or more angularly deflected laser beams with the same intensities. The memory may include instructions to produce two or more angularly deflected laser beams with different intensities, such as 3 or more, such as 4 or more, such as 5 or more, such as 10 or more, such as 25 or more, such as 50 or more and including memory may include instructions to produce 100 or more angularly deflected laser beams with different intensities.

The controller may have a processor having memory operably coupled to the processor such that the memory includes instructions stored thereon, which when executed by the processor, cause the processor to produce an output laser beam having increasing intensity from the edges to the center of the output laser beam along the horizontal axis. The intensity of the angularly deflected laser beam at the center of the output beam may range from 0.1% to about 99% of the intensity of the angularly deflected laser beams at the edge of the output laser beam along the horizontal axis, such as from 0.5% to about 95%, such as from 1% to about 90%, such as from about 2% to about 85%, such as from about 3% to about 80%, such as from about 4% to about 75%, such as from about 5% to about 70%, such as from about 6% to about 65%, such as from about 7% to about 60%, such as from about 8% to about 55% and including from about 10% to about 50% of the intensity of the angularly deflected laser beams at the edge of the output laser beam along the horizontal axis. The controller may have a processor having memory operably coupled to the processor such that the memory includes instructions stored thereon, which when executed by the processor, cause the processor to produce an output laser beam having an increasing intensity from the edges to the center of the output laser beam along the horizontal axis. The intensity of the angularly deflected laser beam at the edges of the output beam may range from 0.1% to about 99% of the intensity of the angularly deflected laser beams at the center of the output laser beam along the horizontal axis, such as from 0.5% to about 95%, such as from 1% to about 90%, such as from about 2% to about 85%, such as from about 3% to about 80%, such as from about 4% to about 75%, such as from about 5% to about 70%, such as from about 6% to about 65%, such as from about 7% to about 60%, such as from about 8% to about 55% and including from about 10% to about 50% of the intensity of the angularly deflected laser beams at the center of the output laser beam along the horizontal axis. The controller may have a processor having memory operably coupled to the processor such that the memory includes instructions stored thereon, which when executed by the processor, cause the processor to produce an output laser beam having an intensity profile with a Gaussian distribution along the horizontal axis. In still other embodiments, the controller has a processor having memory operably coupled to the processor such that the memory includes instructions stored thereon, which when executed by the processor, cause the processor to produce an output laser beam having a top hat intensity profile along the horizontal axis.

Light beam generators of interest may be configured to produce angularly deflected laser beams in the output laser beam that are spatially separated. Depending on the applied radiofrequency drive signals and desired irradiation profile of the output laser beam, the angularly deflected laser beams may be separated by 0.001 µm or more, such as by 0.005 µm or more, such as by 0.01 µm or more, such as by 0.05 µm or more, such as by 0.1 µm or more, such as by 0.5 µm or more, such as by 1 µm or more, such as by 5 µm or more, such as by 10 µm or more, such as by 100 µm or more, such as by 500 µm or more, such as by 1000 µm or more and including by 5000 µm or more. The system can be configured to produce angularly deflected laser beams in the output laser beam that overlap, such as with an adjacent angularly deflected laser beam along a horizontal axis of the output laser beam. The overlap between adjacent angularly deflected laser beams (such as overlap of beam spots) may be an overlap of 0.001 µm or more, such as an overlap of 0.005 µm or more, such as an overlap of 0.01 µm or more, such as an overlap of 0.05 µm or more, such as an overlap of 0.1 µm or more, such as an overlap of 0.5 µm or more, such as an overlap of 1 µm or more, such as an overlap of 5 µm or more, such as an overlap of 10 µm or more and including an overlap of 100 µm or more.

Light beam generators may be configured to generate two or more beams of frequency shifted light include laser excitation modules as described in U.S. Patent Nos. 9,423,353; 9,784,661; 9,983,132; 10,006,852; 10,036,699; 10,078,045; 10,222,316; 10,288,546; 10,324,019; 10,408,758; 10,451,538; 10,620,111; 10,684,211; 10,845,295; 10,935,482; 10,935,485; 11,105,728; 11,280,718; 11,327,016; 11,366,052; 11,371,937; 11,692,926; 11,630,053; 11,774,343; 11,940,369; and 11,946,851.

In addition, flow cytometers include a detector configured to collect light emitted by the irradiated particles. The light detectors are configured to detect particle-modulated light conveyed by the fiber optic light collection elements and generate signals based on a characteristic of that light (e.g., intensity). For example, the one or more particle-modulated light detector(s) may include one or more side-scattered light detectors for detecting side-scatter wavelengths of light (i.e., light refracted and reflected from the surfaces and internal structures of the particle). Flow cytometers may include a single side-scattered light detector. Flow cytometers may include multiple side-scattered light detectors, such as 2 or more, such as 3 or more, such as 4 or more, and including 5 or more.

Any convenient detector for detecting collected light may be used in the side-scattered light detector described herein. Detectors of interest may include, but are not limited to, optical sensors or detectors, such as active-pixel sensors (APSs), avalanche photodiodes, image sensors, charge-coupled devices (CCDs), intensified charge-coupled devices (ICCDs), light emitting diodes, photon counters, bolometers, pyroelectric detectors, photoresistors, photovoltaic cells, photodiodes, photomultiplier tubes (PMTs), phototransistors, quantum dot photoconductors or photodiodes and combinations thereof, among other detectors. The collected light can be measured with a charge-coupled device (CCD), semiconductor charge-coupled devices (CCD), active pixel sensors (APS), complementary metal-oxide semiconductor (CMOS) image sensors or N-type metal-oxide semiconductor (NMOS) image sensors. The detector can be a photomultiplier tube, such as a photomultiplier tube having an active detecting surface area of each region that ranges from 0.01 cm² to 10 cm², such as from 0.05 cm² to 9 cm², such as from 0.1 cm² to 8 cm², such as from 0.5 cm² to 7 cm² and including from 1 cm² to 5 cm².

The subject flow cytometers may also include a fluorescent light detector configured to detect one or more fluorescent wavelengths of light. In other embodiments, flow cytometers include multiple fluorescent light detectors such as 2 or more, such as 3 or more, such as 4 or more, 5 or more, 10 or more, 15 or more, and including 20 or more.

Any convenient detector for detecting collected light may be used in the fluorescent light detector described herein. Detectors of interest may include, but are not limited to, optical sensors or detectors, such as active-pixel sensors (APSs), avalanche photodiodes, image sensors, charge-coupled devices (CCDs), intensified charge-coupled devices (ICCDs), light emitting diodes, photon counters, bolometers, pyroelectric detectors, photoresistors, photovoltaic cells, photodiodes, photomultiplier tubes (PMTs), phototransistors, quantum dot photoconductors or photodiodes and combinations thereof, among other detectors. The collected light can be measured with a charge-coupled device (CCD), semiconductor charge-coupled devices (CCD), active pixel sensors (APS), complementary metal-oxide semiconductor (CMOS) image sensors or N-type metal-oxide semiconductor (NMOS) image sensors. The detector can be a photomultiplier tube, such as a photomultiplier tube having an active detecting surface area of each region that ranges from 0.01 cm² to 10 cm², such as from 0.05 cm² to 9 cm², such as from, such as from 0.1 cm² to 8 cm², such as from 0.5 cm² to 7 cm² and including from 1 cm² to 5 cm².

Where the subject flow cytometers include multiple fluorescent light detectors, each fluorescent light detector may be the same, or the collection of fluorescent light detectors may be a combination of different types of detectors. For example, where the subject flow cytometers include two fluorescent light detectors, the first fluorescent light detector can be a CCD-type device and the second fluorescent light detector (or imaging sensor) can be a CMOS-type device. Both the first and second fluorescent light detectors can be CCD-type devices. Both the first and second fluorescent light detectors can be CMOS-type devices. The first fluorescent light detector can be a CCD-type device and the second fluorescent light detector is a photomultiplier tube (PMT). The first fluorescent light detector can be a CMOS-type device and the second fluorescent light detector can be a photomultiplier tube. Both the first and second fluorescent light detectors can be photomultiplier tubes.

Fluorescent light detectors of interest may be configured to measure collected light at one or more wavelengths, such as at 2 or more wavelengths, such as at 5 or more different wavelengths, such as at 10 or more different wavelengths, such as at 25 or more different wavelengths, such as at 50 or more different wavelengths, such as at 100 or more different wavelengths, such as at 200 or more different wavelengths, such as at 300 or more different wavelengths and including measuring light emitted by a sample in the flow stream at 400 or more different wavelengths.2 or more detectors in the modules as described herein may be configured to measure the same or overlapping wavelengths of collected light.

Fluorescent light detectors of interest can be configured to measure collected light over a range of wavelengths (e.g., 200 nm - 1000 nm). Detectors of interest can be configured to collect spectra of light over a range of wavelengths. For example, flow cytometers may include one or more detectors configured to collect spectra of light over one or more of the wavelength ranges of 200 nm - 1000 nm. Detectors of interest can be configured to measure light emitted by a sample in the flow stream at one or more specific wavelengths. For example, modules may include one or more detectors configured to measure light at one or more of 450 nm, 518 nm, 519 nm, 561 nm, 578 nm, 605 nm, 607 nm, 625 nm, 650 nm, 660 nm, 667 nm, 670 nm, 668 nm, 695 nm, 710 nm, 723 nm, 780 nm, 785 nm, 647 nm, 617 nm and any combinations thereof.One or more detectors may be configured to be paired with specific fluorophores, such as those used with the sample in a fluorescence assay.

Flow cytometers may include any suitable mechanism(s) for providing sheath fluid and sample fluid to the sample fluid input coupler and sheath fluid input coupler. For example, the sample fluid input coupler may be fluidically connected to a sample fluid line (e.g., tubing) fluidically connected to a sample fluid reservoir. Similarly, the sheath fluid input coupler may be fluidically connected to a sheath fluid line fluidically connected to a sheath fluid reservoir. Similarly, flow cytometers may include any suitable mechanism(s) for managing waste from the flow stream. The fluidic output coupler may be fluidically connected to a waste line fluidically connected to a waste reservoir. Fluid management systems that may be adapted for use in the subject flow cytometers are provided in U.S. Patent Application Publication No. 2022/0341838.

Suitable flow cytometry systems may include, but are not limited to those described in Ormerod (ed.), Flow Cytometry: A Practical Approach, Oxford Univ. Press (1997); Jaroszeski et al. (eds.), Flow Cytometry Protocols, Methods in Molecular Biology No. 91, Humana Press (1997); Practical Flow Cytometry, 3rd ed., Wiley-Liss (1995); Virgo, et al. (2012) Ann Clin Biochem. Jan;49(pt 1):17-28; Linden, et. al., Semin Throm Hemost. 2004 Oct;30(5):502-11; Alison, et al. J Pathol, 2010 Dec; 222(4):335-344; and Herbig, et al. (2007) Crit Rev Ther Drug Carrier Syst. 24(3):203-255. In certain instances, flow cytometry systems of interest include BD Biosciences FACSCanto^{™} flow cytometer, BD Biosciences FACSCanto^{™} II flow cytometer, BD Accuri^{™} flow cytometer, BD Accuri^{™} C6 Plus flow cytometer, BD Biosciences FACSCelesta^{™} flow cytometer, BD Biosciences FACSLyric^{™} flow cytometer, BD Biosciences FACSVerse^{™} flow cytometer, BD Biosciences FACSymphony^{™} flow cytometer, BD Biosciences LSRFortessa^{™} flow cytometer, BD Biosciences LSRFortessa^{™} X-20 flow cytometer, BD Biosciences FACSPresto^{™} flow cytometer, BD Biosciences FACSVia^{™} flow cytometer and BD Biosciences FACSCalibur^{™} cell sorter, a BD Biosciences FACSCount^{™} cell sorter, BD Biosciences FACSLyric^{™} cell sorter, BD Biosciences Via^{™} cell sorter, BD Biosciences Influx^{™} cell sorter, BD Biosciences Jazz^{™} cell sorter, BD Biosciences Aria^{™} cell sorter, BD Biosciences FACSAria^{™} II cell sorter, BD Biosciences FACSAria^{™} III cell sorter, BD Biosciences FACSAria^{™} Fusion cell sorter and BD Biosciences FACSMelody^{™} cell sorter, BD Biosciences FACSymphony^{™} S6 cell sorter, BD Biosciences FACSDiscover^{™} cell sorter, or the like.

The subject system can be a flow cytometric systems, such as described in U.S. Patent Nos. 10,663,476; 10,620,111; 10,613,017; 10,605,713; 10,585,031; 10,578,542; 10,578,469; 10,481,074; 10,302,545; 10,145,793; 10,113,967; 10,006,852; 9,952,076; 9,933,341; 9,726,527; 9,453,789; 9,200,334; 9,097,640; 9,095,494; 9,092,034; 8,975,595; 8,753,573; 8,233,146; 8,140,300; 7,544,326; 7,201,875; 7,129,505; 6,821,740; 6,813,017; 6,809,804; 6,372,506; 5,700,692; 5,643,796; 5,627,040; 5,620,842; 5,602,039; 4,987,086; 4,498,766.

The flow cytometer can be configured as an imaging flow cytometer. For example, the subject systems can be a flow cytometry system configured for imaging particles in a flow stream by fluorescence imaging using radiofrequency tagged emission (FIRE), such as described in Diebold, et al. Nature Photonics Vol. 7(10); 806-810 (2013) as well as described in U.S. Patent Nos. 9,423,353; 9,784,661; 9,983,132; 10,006,852; 10,036,699; 10,078,045; 10,222,316; 10,288,546; 10,324,019; 10,408,758; 10,451,538; 10,620,111; 10,684,211; 10,845,295; 10,935,482; 10,935,485; 11,105,728; 11,280,718; 11,327,016; 11,366,052; 11,371,937; 11,692,926; 11,630,053; 11,774,343; 11,940,369; and 11,946,851. Where the flow cytometer is a particle sorter, the particle sorter is an image enabled particle sorter. Image enabled particle sorters are described in U.S. Patent Nos. 10,324,019; 10,620,111; 11,105,728; and 11,774,343; as well as U.S. Patent Application Nos. 18/537,103; 18/657,618; 18,657,623 and 18/657,633.

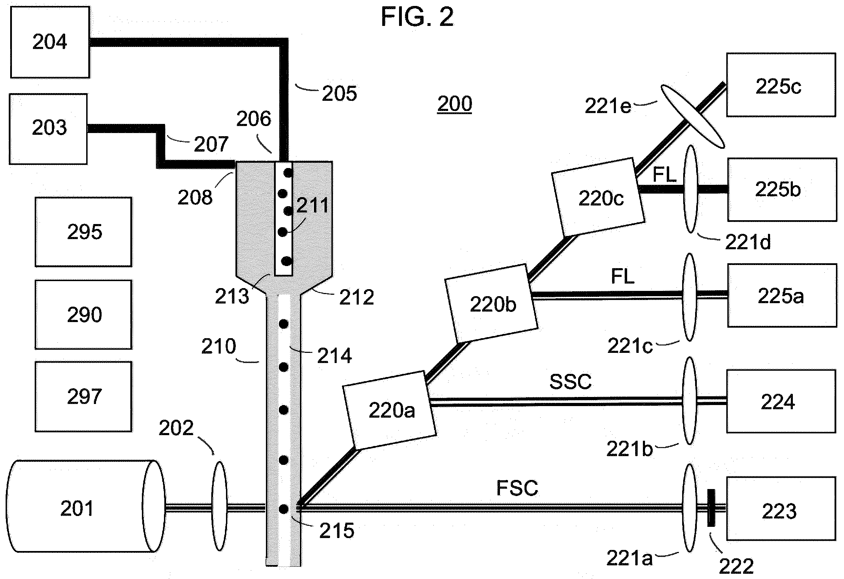

**FIG. 2** shows a system 200 for flow cytometry in accordance with the present disclosure. System 200 includes a laser 201 configured to irradiate particles 211 in flow stream 214 at interrogation point 215 within flow cell 210. While the example of **FIG. 2** shows a single laser, it is understood that multiple lasers could also be used. The laser beam from laser 201 is directed to focusing lens 202 which focuses the beam onto the portion of a fluid stream where particles 211 of a sample are located, within the flow cell 210. The flow cell 210 is part of a fluidics system which directs particles, typically one at a time, in a stream to the focused laser beam for interrogation. Alternatively, where the flow cytometer is a stream-in-air cytometer, a nozzle top may be employed.

As shown in **FIG. 2****,** flow cell 210 is fluidically connected to sheath fluid reservoir 203 comprising a sheath fluid and sample fluid reservoir 204 comprising a sample fluid. Sheath fluid from sheath fluid reservoir 203 is provided to at least one sheath fluid injection port 208 via conduit (i.e., sheath fluid line) 207. In addition, sample fluid containing particles 211 from sample fluid reservoir 204 is provided to sample injection port 206 via conduit (i.e., sample fluid line) 205. Sample injection port 206 is fluidically connected to sample injector 213 (e.g., sample injection needle) which is configured to introduce particles 211 into the interior of flow cell 210. Particles 211 are hydrodynamically focused via sheath fluid entering from sheath fluid injection port 208 such that flow stream 214 forms downstream of tapered portion 212 of flow cell 210. Particles emitting at the distal end of flow cell 210 may be disposed of and/or collected via any suitable protocol. For example, depending on the type of flow cytometry being performed, particles may be collected at the distal end of flow cell 210, e.g., via a waste line. Alternatively, particles may be sorted.

The light from the laser beam(s) interacts with the particles 211 in the sample by diffraction, refraction, reflection, scattering, and absorption with re-emission at various different wavelengths depending on the characteristics of the particle such as its size, internal structure, and the presence of one or more fluorescent molecules attached to or naturally present on or in the particle. The fluorescence emissions as well as the diffracted light, refracted light, reflected light, and scattered light may be routed to one or more detectors. In particular, forward scattered light (FSC) is routed to forward-scattered light detector 223. The forward-scattered light detector 223 is positioned slightly off axis from the direct beam through the flow cell 210 and is configured to detect diffracted light, the excitation light that travels through or around the particle in mostly a forward direction. The intensity of the light detected by the forward-scattered light detector 223 is dependent on the overall size of the particle. The forward-scatter detector can include, e.g., a photodiode. Positioned between forward-scattered light detector 223 are optical filter 221a and scatter bar 222. Optical filter 221a may be configured to filter out at least one wavelength of non-FSC light, while scatter bar 222 may be configured to prevent the incident beam from laser 201 (i.e., non-scattered light) from being detected by forward-scattered light detector 223.

In addition, side-scattered light (SSC) is detected by side-scattered light detector 224. In other words, side-scattered light detector 224 is configured to detect refracted and reflected light from the surfaces and internal structures of the particles 211 that tend to increase with increasing particle complexity of structure. In the example of **FIG. 2****,** flow cytometer 200 includes dichroic mirror 220a configured to reflect SSC light to side-scattered light detector 224 while passing non-SSC (e.g., fluorescent) light. Optical filter 221b is configured to prevent at least one wavelength of non-SSC light from being detected by side-scattered light detector 224. Also shown are fluorescent light detectors 225a-225c which are each configured to detect different wavelengths of fluorescent light. For example, dichroic mirror 220b may be configured to reflect fluorescent light (FL) corresponding to a first wavelength (or range of wavelengths) to fluorescent light detector 225a while passing other wavelengths of light. Optical filter 221c may be configured to prevent at least one wavelength of light that does not correspond to the first wavelength (or range of wavelengths) from being detected by fluorescent light detector 225a. Similarly, dichroic mirror 220c is configured to reflect FL light corresponding to a second wavelength (or range of wavelengths) to fluorescent light detector 225b while passing a third wavelength of light (or range of wavelengths) for detection by fluorescent light detector 225c. Optical filter 221d is configured to prevent at least one wavelength of light that does not correspond to the second wavelength (or range of wavelengths) from being detected by fluorescent light detector 225b. In addition, Optical filter 221e is configured to prevent at least one wavelength of light that does not correspond to the third wavelength (or range of wavelengths) from being detected by fluorescent light detector 225c.

One of skill in the art will recognize that a flow cytometer in accordance with an embodiment of the present disclosure is not limited to the flow cytometer depicted in **FIG. 2****,** but can include any flow cytometer known in the art. For example, a flow cytometer may have any number of lasers, beam splitters, filters, and detectors at various wavelengths and in various different configurations. For example, while the embodiment of **FIG. 2** shows 3 fluorescent light detectors for illustrative purposes, it is understood that any suitable number of fluorescent light detectors may be employed.

In operation, cytometer operation is controlled by a controller/processor 290, and the measurement data from the detectors can be stored in the memory 295 and processed by the controller/processor 290. Although not shown explicitly, the controller/processor 290 is coupled to the detectors to receive the output signals therefrom, and may also be coupled to electrical and electromechanical components of the flow cytometer to control the laser 201, fluid flow parameters, and the like. Input/output (I/O) capabilities 297 may be provided also in the system. The memory 295, controller/processor 290, and I/O 297 may be entirely provided as an integral part of the flow cytometer. In such an embodiment, a display may also form part of the I/O capabilities 297 for presenting experimental data to users of the cytometer 200. Alternatively, some or all of the memory 295 and controller/processor 290 and I/O capabilities may be part of one or more external devices such as a general purpose computer. Some or all of the memory 295 and controller/processor 290 can be in wireless or wired communication with the cytometer 210. The controller/processor 290 in conjunction with the memory 295 and the I/O 297 can be configured to perform various functions related to the preparation and analysis of a flow cytometer experiment.

Different fluorescent molecules in a fluorochrome panel used for a flow cytometer experiment will emit light in their own characteristic wavelength bands. The particular fluorescent labels used for an experiment and their associated fluorescent emission bands may be selected to generally coincide with the filter windows of the detectors. The I/O 297 can be configured to receive data regarding a flow cytometer experiment having a panel of fluorescent labels and a plurality of cell populations having a plurality of markers, each cell population having a subset of the plurality of markers. The I/O 297 can also be configured to receive biological data assigning one or more markers to one or more cell populations, marker density data, emission spectrum data, data assigning labels to one or more markers, and cytometer configuration data. Flow cytometer experiment data, such as label spectral characteristics and flow cytometer configuration data can also be stored in the memory 295. The controller/processor 290 can be configured to evaluate one or more assignments of labels to markers.

The subject systems can be a particle sorting system configured to sort particles with an enclosed particle sorting module, such as those described in U.S. Patent Publication No. 2017/0299493, filed on March 28, 2017. Particles (e.g., cells) of the sample can be sorted using a sort decision module having a plurality of sort decision units, such as those described in U.S. Patent Publication No. 2020/0256781, filed on December 23, 2019. The present disclosure for sorting components of a sample include a particle sorting module having deflection plates, such as described in U.S. Patent Publication No. 2017/0299493, filed on March 28, 2017.

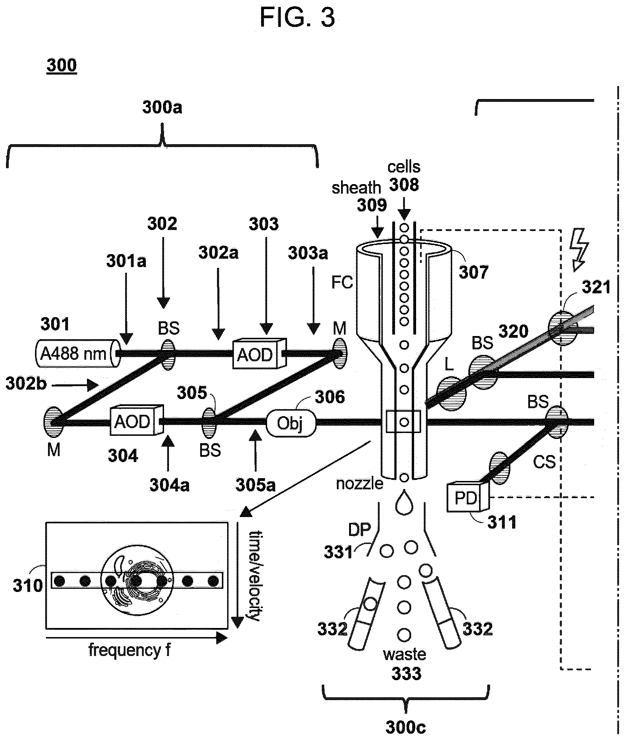

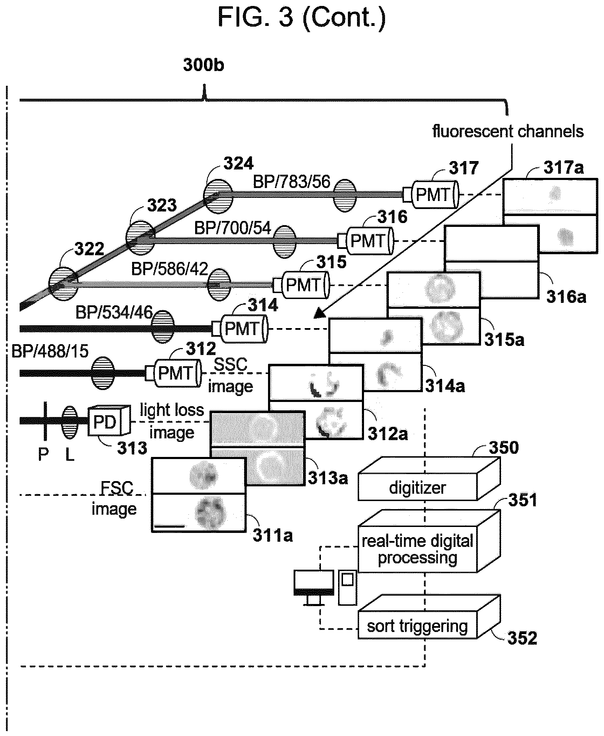

The system can be a fluorescence imaging using radiofrequency tagged emission image-enabled particle sorter, such as depicted in **FIG. 3****.** Particle sorter 300 includes a light irradiation component 300a which includes light source 301 (e.g., 488 nm laser) which generates output beam of light 301a that is split with beamsplitter 302 into beams 302a and 302b. Light beam 302a is propagated through acousto-optic device (e.g., an acousto-optic deflector, AOD) 303 to generate an output beam 303a having one or more angularly deflected beams of light. In some instances, output beam 303a generated from acousto-optic device 303 includes a local oscillator beam and a plurality of radiofrequency comb beams. Light beam 302b is propagated through acousto-optic device (e.g., an acousto-optic deflector, AOD) 304 to generate an output beam 304a having one or more angularly deflected beams of light. In some instances, output beam 304a generated from acousto-optic device 304 includes a local oscillator beam and a plurality of radiofrequency comb beams. Output beams 303a and 304a generated from acousto-optic devices 303 and 304, respectively are combined with beamsplitter 305 to generate output beam 305a which is conveyed through an optical component 306 (e.g., an objective lens) to irradiate particles in flow cell 307. Acousto-optic device 303 (AOD) may split a single laser beam into an array of beamlets, each having different optical frequency and angle. Second AOD 304 tunes the optical frequency of a reference beam, which is then overlapped with the array of beamlets at beam combiner 305. , The light irradiation system having a light source and acousto-optic device can also include those described in Schraivogel, et al. ("High-speed fluorescence image-enabled cell sorting" Science (2022), 375 (6578): 315-320) and United States Patent Publication No. 2021/0404943.

Output beam 305a irradiates sample particles 308 propagating through flow cell 307 (e.g., with sheath fluid 309) at irradiation region 310. As shown in irradiation region 310, a plurality of beams (e.g., angularly deflected radiofrequency shifted beams of light depicted as dots across irradiation region 310) overlaps with a reference local oscillator beam (depicted as the shaded line across irradiation region 310). Due to their differing optical frequencies, the overlapping beams exhibit a beating behavior, which causes each beamlet to carry a sinusoidal modulation at a distinct frequency f₁₋ₙ.

Light from the irradiated sample is conveyed to light detection system 300b that includes a plurality of photodetectors. Light detection system 300b includes forward scattered light photodetector 311 for generating forward scatter images 311a and a side scattered light photodetector 312 for generating side scatter images 312a. Light detection system 300b also includes brightfield photodetector 313 for generating light loss images 313a. Forward scatter detector 311 and side scatter detector 312 can be photodiodes (e.g., avalanche photodiodes, APDs). Brightfield photodetector 313 can be a photomultiplier tube (PMT). Fluorescence from the irradiated sample may also be detected with fluorescence photodetectors 314-317. Photodetectors 314-317 may be photomultiplier tubes. Light from the irradiated sample is directed to the side scatter detection channel 312 and fluorescence detection channels 314-317 through beamsplitter 320. Light detection system 300b includes bandpass optical components 321, 322, 323 and 324 (e.g., dichroic mirrors) for propagating predetermined wavelength of light to photodetectors 314-317. Optical component 321 can be a 534 nm/40 nm bandpass. Optical component 322 can be a 586 nm/42 nm bandpass. Optical component 323 can be a 700 nm/54 nm bandpass. Optical component 324 can be a 783 nm/56 nm bandpass. The first number represents the center of a spectral band. The second number provides a range of the spectral band. Thus, a 510/20 filter extends 10 nm on each side of the center of the spectral band, or from 500 nm to 520 nm.

Data signals generated in response to light detected in scattered light detection channels 311 and 312, brightfield light detection channel 313 and fluorescence detection channels 314-317 are processed by real-time digital processing with processors 350 and 351. Images 311a-317a can be generated in each light detection channel based on the data signals generated in processors 350 and 351. Image-enabled sorting is performed in response to a sort signal generated in sort trigger 352. Sorting component 300c includes deflection plates 331 for deflecting particles into sample containers 332 or to waste stream 333. In some instances, sort component 300c is configured to sort particles with an enclosed particle sorting module, such as those described in U.S. Patent Publication No. 2017/0299493, filed on March 28, 2017. Sorting component 300c may include a sort decision module having a plurality of sort decision units, such as those described in U.S. Patent Publication No. 2020/0256781..

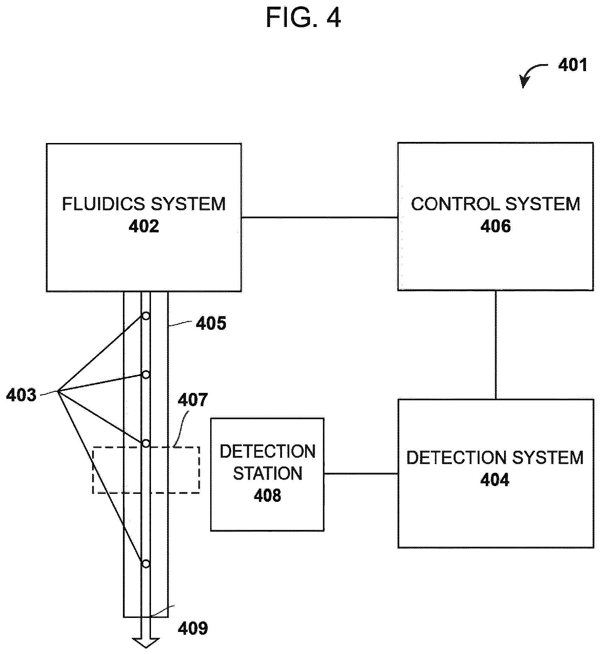

The system can be a particle analyzer where the particle analysis system 401 **(****FIG. 4****)** can be used to analyze and characterize particles, with or without physically sorting the particles into collection vessels. **FIG. 4** shows a functional block diagram of a particle analysis system for computational based sample analysis and particle characterization. The particle analysis system 401 can be a flow system. The particle analysis system 401 includes a fluidics system 402. The fluidics system 402 can include or be coupled with a sample tube 405 and a moving fluid column within the sample tube in which particles 403 (e.g. cells) of a sample move along a common sample path 409.

The particle analysis system 401 includes a detection system 404 configured to collect a signal from each particle as it passes one or more detection stations along the common sample path. A detection station 408 generally refers to a monitored area 407 of the common sample path. Detection can, in some implementations, include detecting light or one or more other properties of the particles 403 as they pass through a monitored area 407. In **FIG. 4****,** one detection station 408 with one monitored area 407 is shown. The particle analysis system 401 can include multiple detection stations. Furthermore, some detection stations can monitor more than one area.

Each signal is assigned a signal value to form a data point for each particle. As described above, this data can be referred to as event data. The data point can be a multidimensional data point including values for respective properties measured for a particle. The detection system 404 is configured to collect a succession of such data points in a first-time interval.

The particle analysis system 401 can also include a control system 406. The control system 406 can include one or more processors, an amplitude control circuit and/or a frequency control circuit. The control system shown can be operationally associated with the fluidics system 402. The control system can be configured to generate a calculated signal frequency for at least a portion of the first-time interval based on a Poisson distribution and the number of data points collected by the detection system 404 during the first time interval. The control system 406 can be further configured to generate an experimental signal frequency based on the number of data points in the portion of the first time interval. The control system 406 can additionally compare the experimental signal frequency with that of a calculated signal frequency or a predetermined signal frequency.

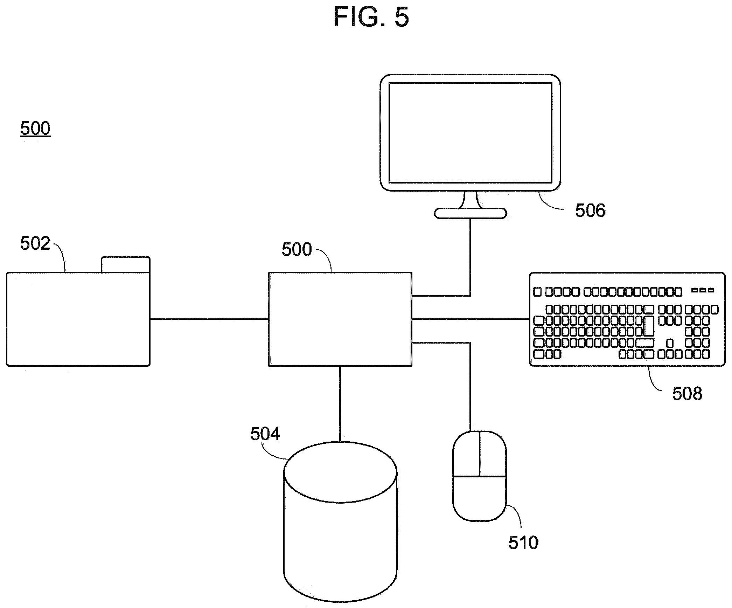

**FIG. 5** shows a functional block diagram for one example of a particle analyzer control system, such as an analytics controller (i.e., processor) 500, for analyzing and displaying biological events. An analytics controller 500 can be configured to implement a variety of processes for controlling graphic display of biological events.

A particle analyzer or sorting system 502 can be configured to acquire biological event data. For example, a flow cytometer can generate flow cytometric event data. The particle analyzer 502 can be configured to provide biological event data to the analytics controller 500. A data communication channel can be included between the particle analyzer or sorting system 502 and the analytics controller 500. The biological event data can be provided to the analytics controller 500 via the data communication channel.

The analytics controller 500 can be configured to receive biological event data from the particle analyzer or sorting system 502. The biological event data received from the particle analyzer or sorting system 502 can include flow cytometric event data. The analytics controller 500 can be configured to provide a graphical display including a first plot of biological event data to a display device 506. The analytics controller 500 can be further configured to render a region of interest as a gate around a population of biological event data shown by the display device 506, overlaid upon the first plot, for example. The gate can be a logical combination of one or more graphical regions of interest drawn upon a single parameter histogram or bivariate plot. The display can be used to display particle parameters or saturated detector data.

The analytics controller 500 can be further configured to display the biological event data on the display device 506 within the gate differently from other events in the biological event data outside of the gate. For example, the analytics controller 500 can be configured to render the color of biological event data contained within the gate to be distinct from the color of biological event data outside of the gate. The display device 506 can be implemented as a monitor, a tablet computer, a smartphone, or other electronic device configured to present graphical interfaces.

The analytics controller 500 can be configured to receive a gate selection signal identifying the gate from a first input device. For example, the first input device can be implemented as a mouse 510. The mouse 510 can initiate a gate selection signal to the analytics controller 500 identifying the gate to be displayed on or manipulated via the display device 506 (e.g., by clicking on or in the desired gate when the cursor is positioned there). In some implementations, the first device can be implemented as the keyboard 508 or other means for providing an input signal to the analytics controller 500 such as a touchscreen, a stylus, an optical detector, or a voice recognition system. Some input devices can include multiple inputting functions. In such implementations, the inputting functions can each be considered an input device. For example, as shown in **FIG. 5****,** the mouse 510 can include a right mouse button and a left mouse button, each of which can generate a triggering event.

The triggering event can cause the analytics controller 500 to alter the manner in which the data is displayed, which portions of the data is actually displayed on the display device 506, and/or provide input to further processing such as selection of a population of interest for particle sorting.

The analytics controller 500 can be configured to detect when gate selection is initiated by the mouse 510. The analytics controller 500 can be further configured to automatically modify plot visualization to facilitate the gating process. The modification can be based on the specific distribution of biological event data received by the analytics controller 500.

The analytics controller 500 can be connected to a storage device 504. The storage device 504 can be configured to receive and store biological event data from the analytics controller 500. The storage device 504 can also be configured to receive and store flow cytometric event data from the analytics controller 500. The storage device 504 can be further configured to allow retrieval of biological event data, such as flow cytometric event data, by the analytics controller 500.

A display device 506 can be configured to receive display data from the analytics controller 500. The display data can comprise plots of biological event data and gates outlining sections of the plots. The display device 506 can be further configured to alter the information presented according to input received from the analytics controller 500 in conjunction with input from the particle analyzer 502, the storage device 504, the keyboard 508, and/or the mouse 510.

In some implementations, the analytics controller 500 can generate a user interface to receive example events for sorting. For example, the user interface can include a control for receiving example events or example images. The example events or images or an example gate can be provided prior to collection of event data for a sample, or based on an initial set of events for a portion of the sample.

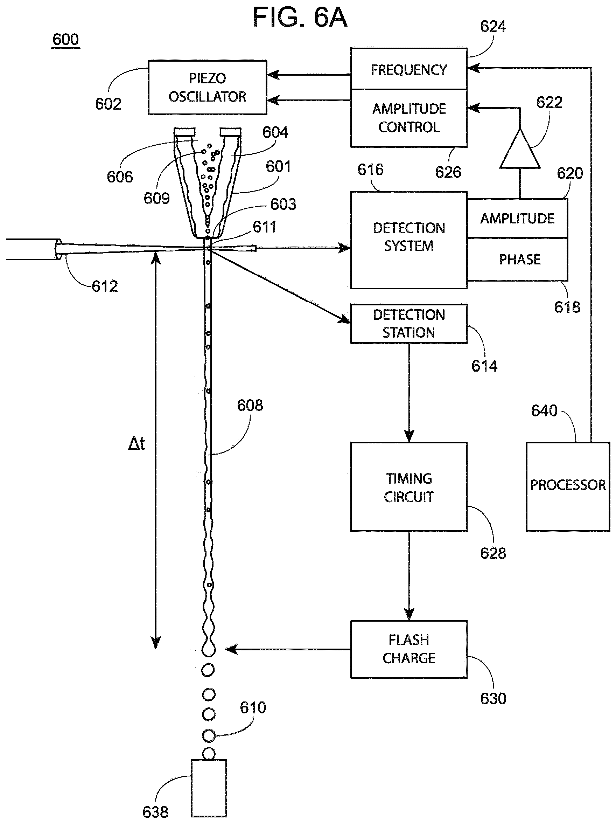

**FIG. 6A** is a schematic drawing of a particle sorter system 600 (e.g., the particle analyzer or sorting system 502) in accordance with the present disclosure. The particle sorter system 600 can be a cell sorter system. As shown in **FIG. 6A****,** a drop formation transducer 602 (e.g., piezo-oscillator) is coupled to a fluid conduit 601, which can be coupled to, can include, or can be, a nozzle 603. Within the fluid conduit 601, sheath fluid 604 hydrodynamically focuses a sample fluid 606 comprising particles 609 into a moving fluid column 608 (e.g., a stream). Within the moving fluid column 608, particles 609 (e.g., cells) are lined up in single file to cross a monitored area 611 (e.g., where laser-stream intersect), irradiated by an irradiation source 612 (e.g., a laser). Vibration of the drop formation transducer 602 causes moving fluid column 608 to break into a plurality of drops 610, some of which contain particles 609.

In operation, a detection station 614 (e.g., an event detector) identifies when a particle of interest (or cell of interest) crosses the monitored area 611. Detection station 614 feeds into a timing circuit 628, which in turn feeds into a flash charge circuit 630. At a drop break off point, informed by a timed drop delay (Δt), a flash charge can be applied to the moving fluid column 608 such that a drop of interest carries a charge. The drop of interest can include one or more particles or cells to be sorted. The charged drop can then be sorted by activating deflection plates (not shown) to deflect the drop into a vessel such as a collection tube or a multi- well or microwell sample plate where a well or microwell can be associated with drops of particular interest. As shown in **FIG**. **6A****,** the drops can be collected in a drain receptacle 638.

A detection system 616 (e.g., a drop boundary detector) serves to automatically determine the phase of a drop drive signal when a particle of interest passes the monitored area 611. An exemplary drop boundary detector is described in U.S. Pat. No. 7,679,039. The detection system 616 allows the instrument to accurately calculate the place of each detected particle in a drop. The detection system 616 can feed into an amplitude signal 620 and/or phase 618 signal, which in turn feeds (via amplifier 622) into an amplitude control circuit 626 and/or frequency control circuit 624. The amplitude control circuit 626 and/or frequency control circuit 624, in turn, controls the drop formation transducer 602. The amplitude control circuit 626 and/or frequency control circuit 624 can be included in a control system.

Sort electronics (e.g., the detection system 616, the detection station 614 and a processor 640) can be coupled with a memory configured to store the detected events and a sort decision based thereon. The sort decision can be included in the event data for a particle. In some implementations, the detection system 616 and the detection station 614 can be implemented as a single detection unit or communicatively coupled such that an event measurement can be collected by one of the detection system 616 or the detection station 614 and provided to the non-collecting element.

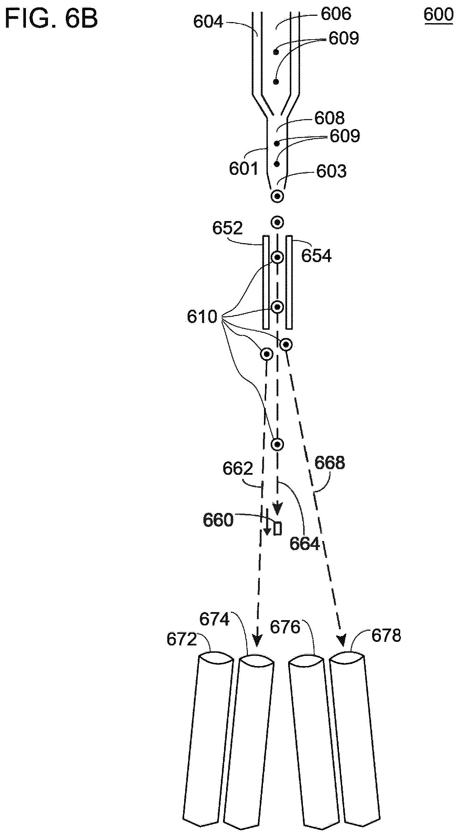

**FIG. 6B** is a schematic drawing of a particle sorter system, in accordance with one embodiment presented herein. The particle sorter system 600 shown in **FIG. 6B****,** includes deflection plates 652 and 654. A charge can be applied via a stream-charging wire in a barb. This creates a stream of droplets 610 containing particles 610 for analysis. The particles can be illuminated with one or more light sources (e.g., lasers) to generate light scatter and fluorescence information. The information for a particle is analyzed such as by sorting electronics or other detection system (not shown in FIG. 6B). The deflection plates 652 and 654 can be independently controlled to attract or repel the charged droplet to guide the droplet toward a destination collection receptacle (e.g., one of 672, 674, 676, or 678). As shown in **FIG. 6B****,** the deflection plates 652 and 654 can be controlled to direct a particle along a first path 662 toward the receptacle 674 or along a second path 668 toward the receptacle 678. If the particle is not of interest (e.g., does not exhibit scatter or illumination information within a specified sort range), deflection plates may allow the particle to continue along a flow path 664. Such uncharged droplets may pass into a waste receptacle such as via aspirator 670.

The sorting electronics can be included to initiate collection of measurements, receive fluorescence signals for particles, and determine how to adjust the deflection plates to cause sorting of the particles. The example shown in **FIG. 6B** includes the BD FACSAria^{™} line of flow cytometers commercially provided by Becton, Dickinson and Company (Franklin Lakes, NJ).

### COMPUTER-CONTROLLED SYSTEMS

Aspects of the disclosure additionally include a computer-controlled system, where the system includes one or more computers for complete automation or partial automation. The system may include a computer having a non-transitory computer readable storage medium with a computer program stored thereon, where the computer program when loaded on the computer includes instructions for performing methods of the disclosure. For example, the computer may be configured to identify measurement uncertainty corresponding to individual particles in the sample, or identify measurement uncertainty for individual parameters of detected light for particles in the sample or may be configured for generating a quality score for each particle based on the measurement uncertainty for each particle.

The system may include a display and operator input device. Operator input devices may, for example, be a keyboard, mouse, or the like. The processing module includes a processor which has access to a memory having instructions stored thereon for performing the steps of the subject methods. The processing module may include an operating system, a graphical user interface (GUI) controller, a system memory, memory storage devices, and input-output controllers, cache memory, a data backup unit, and many other devices. The processor may be a commercially available processor, or it may be one of other processors that are or will become available. The processor executes the operating system and the operating system interfaces with firmware and hardware in a well-known manner, and facilitates the processor in coordinating and executing the functions of various computer programs that may be written in a variety of programming languages, such as Java, Perl, C++, Python, other high level or low level languages, as well as combinations thereof, as is known in the art. The operating system, typically in cooperation with the processor, coordinates and executes functions of the other components of the computer. The operating system also provides scheduling, input-output control, file and data management, memory management, and communication control and related services, all in accordance with known techniques. The processor may include analog electronics which provide feedback control, such as for example negative feedback control.

The system memory may be any of a variety of known or future memory storage devices. Examples include any commonly available random access memory (RAM), magnetic medium such as a resident hard disk or tape, an optical medium such as a read and write compact disc, flash memory devices, or other memory storage device. The memory storage device may be any of a variety of known or future devices, including a compact disk drive, a tape drive, or a diskette drive. Such types of memory storage devices typically read from, and/or write to, a program storage medium (not shown) such as a compact disk. Any of these program storage media, or others now in use or that may later be developed, may be considered a computer program product. As will be appreciated, these program storage media typically store a computer software program and/or data. Computer software programs, also called computer control logic, typically are stored in system memory and/or the program storage device used in conjunction with the memory storage device.

A computer program product is described comprising a computer usable medium having control logic (computer software program, including program code) stored therein. The control logic, when executed by the processor the computer, causes the processor to perform functions described herein. In other embodiments, some functions are implemented primarily in hardware using, for example, a hardware state machine. Implementation of the hardware state machine so as to perform the functions described herein will be apparent to those skilled in the relevant arts.

Memory may be any suitable device in which the processor can store and retrieve data, such as magnetic, optical, or solid-state storage devices (including magnetic or optical disks or tape or RAM, or any other suitable device, either fixed or portable). The processor may include a general-purpose digital microprocessor suitably programmed from a computer readable medium carrying necessary program code. Programming can be provided remotely to processor through a communication channel, or previously saved in a computer program product such as memory or some other portable or fixed computer readable storage medium using any of those devices in connection with memory. For example, a magnetic or optical disk may carry the programming, and can be read by a disk writer/reader. The system of the disclosure may also include programming, e.g., in the form of computer program products, algorithms for use in practicing the methods as described above. Programming according to the present disclosure can be recorded on computer readable media, e.g., any medium that can be read and accessed directly by a computer. Such media include, but are not limited to: magnetic storage media, such as floppy discs, hard disc storage medium, and magnetic tape; optical storage media such as CD-ROM; electrical storage media such as RAM and ROM; portable flash drive; and hybrids of these categories such as magnetic/optical storage media.

The processor may also have access to a communication channel to communicate with a user at a remote location. By remote location is meant the user is not directly in contact with the system and relays input information to an input manager from an external device, such as a computer connected to a Wide Area Network ("WAN"), telephone network, satellite network, or any other suitable communication channel, including a mobile telephone (i.e., smartphone).

The system according to the present disclosure may be configured to include a communication interface. The communication interface may include a receiver and/or transmitter for communicating with a network and/or another device. The communication interface can be configured for wired or wireless communication, including, but not limited to, radio frequency (RF) communication (e.g., RadioFrequency Identification (RFID), Zigbee communication protocols, Wi-Fi, infrared, wireless Universal Serial Bus (USB), Ultra Wide Band (UWB), Bluetooth^{®} communication protocols, and cellular communication, such as code division multiple access (CDMA) or Global System for Mobile communications (GSM).

In one embodiment, the communication interface is configured to include one or more communication ports, e.g., physical ports or interfaces such as a USB port, a USB-C port, an RS-232 port, or any other suitable electrical connection port to allow data communication between the subject systems and other external devices such as a computer terminal (for example, at a physician's office or in hospital environment) that is configured for similar complementary data communication.

The communication interface can be configured for infrared communication, Bluetooth^{®} communication, or any other suitable wireless communication protocol to enable the subject systems to communicate with other devices such as computer terminals and/or networks, communication enabled mobile telephones, personal digital assistants, or any other communication devices which the user may use in conjunction.

The communication interface can be configured to provide a connection for data transfer utilizing Internet Protocol (IP) through a cell phone network, Short Message Service (SMS), wireless connection to a personal computer (PC) on a Local Area Network (LAN) which is connected to the internet, or Wi-Fi connection to the internet at a Wi-Fi hotspot.

The subject system can be configured to wirelessly communicate with a server device via the communication interface, e.g., using a common standard such as 802.11 or Bluetooth^{®} RF protocol, or an IrDA infrared protocol. The server device may be another portable device, such as a smart phone, Personal Digital Assistant (PDA) or notebook computer; or a larger device such as a desktop computer, appliance, etc. The server device may have a display, such as a liquid crystal display (LCD), as well as an input device, such as buttons, a keyboard, mouse or touch-screen.

The communication interface can be configured to automatically or semiautomatically communicate data stored in the subject systems, e.g., in an optional data storage unit, with a network or server device using one or more of the communication protocols and/or mechanisms described above.

Output controllers may include controllers for any of a variety of known display devices for presenting information to a user, whether a human or a machine, whether local or remote. If one of the display devices provides visual information, this information typically may be logically and/or physically organized as an array of picture elements. A graphical user interface (GUI) controller may include any of a variety of known or future software programs for providing graphical input and output interfaces between the system and a user, and for processing user inputs. The functional elements of the computer may communicate with each other via system bus. Some of these communications may be accomplished in alternative embodiments using network or other types of remote communications. The output manager may also provide information generated by the processing module to a user at a remote location, e.g., over the Internet, phone or satellite network, in accordance with known techniques. The presentation of data by the output manager may be implemented in accordance with a variety of known techniques. As some examples, data may include SQL, HTML or XML documents, email or other files, or data in other forms. The data may include Internet URL addresses so that a user may retrieve additional SQL, HTML, XML, or other documents or data from remote sources. The one or more platforms present in the subject systems may be any type of known computer platform or a type to be developed in the future, although they typically will be of a class of computer commonly referred to as servers. However, they may also be a main-frame computer, a workstation, or other computer type. They may be connected via any known or future type of cabling or other communication system including wireless systems, either networked or otherwise. They may be co-located or they may be physically separated. Various operating systems may be employed on any of the computer platforms, possibly depending on the type and/or make of computer platform chosen. Appropriate operating systems include Windows^{□} NT^{□}, Windows^{□} XP, Windows^{□} 7, Windows^{□} 8, Windows^{□} 10, iOS^{□}, macOS^{□}, Linux^{□}, Ubuntu^{□}, Fedora^{□}, OS/400^{□}, i5/OS^{□}, IBM i^{□}, Android^{™}, SGI IRIX^{□}, Oracle Solaris^{□} and others.

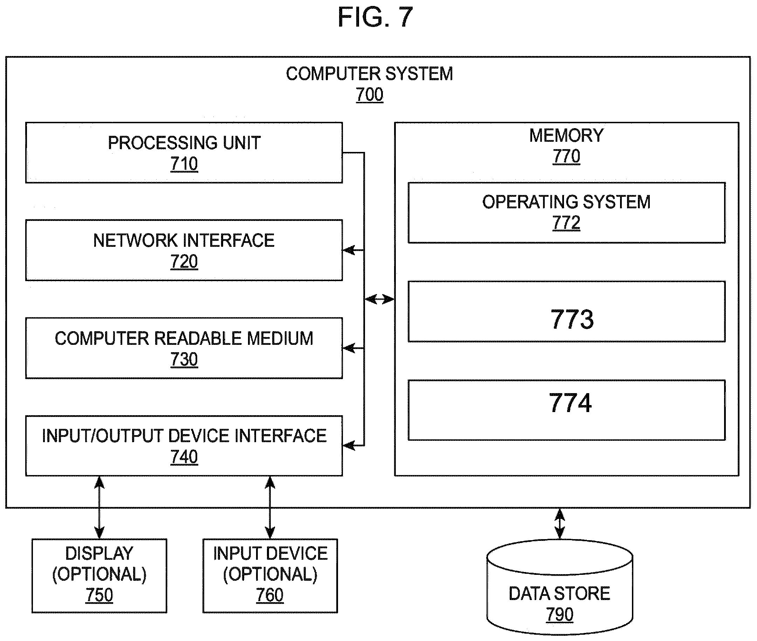

**FIG. 7** depicts a general architecture of an example computing device 700 according to the present disclosure. The general architecture of the computing device 700 depicted in **FIG. 7** includes an arrangement of computer hardware and software components. It is not necessary, however, that all of these generally conventional elements be shown in order to provide an enabling disclosure. As illustrated, the computing device 700 includes a processing unit 710, a network interface 720, a computer readable medium drive 730, an input/output device interface 740, a display 750, and an input device 760, all of which may communicate with one another by way of a communication bus. The network interface 720 may provide connectivity to one or more networks or computing systems. The processing unit 710 may thus receive information and instructions from other computing systems or services via a network. The processing unit 710 may also communicate to and from memory 770 and further provide output information for an optional display 750 via the input/output device interface 740. For example, an analysis software (e.g., data analysis software or program such as FlowJo^{®}) stored as executable instructions in the non-transitory memory of the analysis system can display the flow cytometry event data to a user. The input/output device interface 740 may also accept input from the optional input device 760, such as a keyboard, mouse, digital pen, microphone, touch screen, gesture recognition system, voice recognition system, gamepad, accelerometer, gyroscope, or other input device.

The memory 770 may contain computer program instructions (grouped as modules or components) that the processing unit 710 executes in order to implement one or more embodiments. The memory 770 generally includes RAM, ROM and/or other persistent, auxiliary or non-transitory computer-readable media. The memory 770 may store an operating system 772 that provides computer program instructions for use by the processing unit 710 in the general administration and operation of the computing device 700. Data may be stored in data storage device 790. The memory 770 may further include computer program instructions and other information for implementing aspects of the present disclosure, such as module for identifying measurement uncertainty associated with the detected light 773 or module for recording measurement uncertainty associated with the detected light 774.

### INTEGRATED CIRCUIT DEVICES

The present disclosure also includes an integrated circuit device programmed for identifying measurement uncertainty associated with light detected from a sample as described in the methods detailed above. The integrated circuit devices of interest may include a field programmable gate array (FPGA). Integrated circuit devices may include an application specific integrated circuit (ASIC). The integrated circuit device may include a complex programmable logic device (CPLD). The integrated circuit device can be are configured for practicing practice the subject methods, as described herein.

### KITS

The present disclosure further includes a kit, where the kit includes one or more of the integrated circuit described herein or the system described herein or the non-transitory computer readable recording media described herein. The kit may further include programming for the subject systems, such as in the form of a computer readable medium (e.g., flash drive, USB storage, compact disk, DVD, Blu-ray disk, etc.) or instructions for downloading the programming from an internet web protocol or cloud server.

In addition to the above components, the subject kit may further include instructions. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, and the like. Yet another form of these instructions is a computer readable medium, e.g., diskette, compact disk (CD), portable flash drive, and the like, on which the information has been recorded. Yet another form of these instructions that may be present is a website address which may be used via the internet to access the information at a removed site.

### UTILITY

The disclosure may find use in a variety of applications where it is desirable to analyze and sort particle components in a sample in a fluid medium, such as a biological sample. The system and method described herein find use in flow cytometry characterization of biological samples labelled with fluorescent tags. The system and method can find use in spectroscopy of emitted light. In addition, the subject system and method can find use in increasing the obtainable signal from light collected from a sample (e.g., in a flow stream). The present disclosure finds use in enhancing measurement of light collected from a sample that is irradiated in a flow stream in a flow cytometer. The present disclosure can find use where it is desirable to provide a flow cytometer with improved cell sorting accuracy, enhanced particle collection, particle charging efficiency, more accurate particle charging and enhanced particle deflection during cell sorting.

The disclosure finds use in applications where cells prepared from a biological sample may be desired for research, laboratory testing or for use in therapy. The subject method and device may facilitate obtaining and/ or analyzing individual cells prepared from a target fluidic or tissue biological sample. For example, the subject methods and systems facilitate obtaining cells from fluidic or tissue samples to be used as a research or diagnostic specimen for diseases such as cancer. Likewise, the subject methods and systems may facilitate obtaining cells from fluidic or tissue samples to be used in therapy.

Notwithstanding the appended claims, the disclosure is also defined by the following clauses
Clause 1. A method comprising:
   introducing a sample into a flow cytometer;
   flowing the introduced sample in a flow stream;
   irradiating the sample in the flow stream with a light source;
   detecting light from particles in the sample flowing in the flow stream; and
   identifying measurement uncertainty associated with the detected light.
Clause 2. The method of clause 1, wherein measurement uncertainty is identified corresponding to individual particles in the sample.
Clause 3. The method of any of the previous clauses, wherein detecting light from particles comprises measuring a plurality of parameters of the detected light for particles in the sample.
Clause 4. The method of any of the previous clauses, wherein measurement uncertainty is identified for individual parameters of detected light for particles in the sample.
Clause 5. The method of any of the previous clauses, further comprising: generating event data based on the detected light, wherein event data comprises parameter measurements for particles in the sample.
Clause 6. The method of any of the previous clauses, further comprising:
   spectrally resolving the light detected from particles.
Clause 7. The method of any of the previous clauses, wherein spectrally resolving the light detected from particles produces a plurality of derived parameters.
Clause 8. The method of any of the previous clauses, further comprising:
   generating a plurality of derived parameters for each particle by spectrally resolving the light detected from each particle.
Clause 9. The method of any of the previous clauses, wherein the derived parameters comprise unmixed detected light.
Clause 10. The method of any of the previous clauses, wherein measurement uncertainty is identified for derived parameters.
Clause 11. The method of any of the previous clauses, further comprising:
   identifying a first subset of particles in the sample by applying a first gate to the detected light.
Clause 12. The method of clause 11, wherein measurement uncertainty corresponds to identifying the first subset of particles.
Clause 13. The method of any of the previous clauses, further comprising:
   identifying a plurality of subsets of particles in the sample by applying a corresponding plurality of gates to the detected light.
Clause 14. The method of clause 13, wherein measurement uncertainty corresponds to identifying each of the plurality of subsets of particles.
Clause 15. The method of any of the previous clauses, wherein measurement uncertainty corresponds to gate-level uncertainty.
Clause 16. The method of any of the previous clauses, further comprising:
   generating a quality score (Q score) for each particle based on the measurement uncertainty for each particle.
Clause 17. The method of any of the previous clauses, further comprising:
   recording each event and associated measurement uncertainty for each event.
Clause 18. The method of any of the previous clauses, further comprising:
   recording measurements for each particle and associated measurement uncertainty for each particle.
Clause 19. The method of any of the previous clauses, further comprising:
   recording measurements for a plurality of parameters for each particle and associated measurement uncertainty for each parameter of each particle.
Clause 20. The method of any of the previous clauses, further comprising:
   recording measurement uncertainty, wherein the measurement uncertainty corresponds to measurement uncertainty for one or more of: each recorded parameter; each recorded event; each gate or other classification; or for the sample.
Clause 21. The method of any of the previous clauses, wherein the measurement uncertainty for different parameters measured for particles in the sample comprises a different metric.
Clause 22. The method of any of the previous clauses, wherein the measurement uncertainty corresponds to a binary classification event.
Clause 23. The method of clause 22, wherein the binary classification event corresponds to one or more of: a gating determination or a population membership classification decision.
Clause 24. The method of any of the previous clauses, wherein the measurement uncertainty comprises a metric that quantifies measurement uncertainty.
Clause 25. The method of any of the previous clauses, further comprising: generating an uncertainty score based on the measurement uncertainty.
Clause 26. The method of any of the previous clauses, wherein measurement uncertainty is reflected in an uncertainty score, wherein higher value uncertainty scores indicates greater measurement uncertainty.
Clause 27. The method of any of the previous clauses, further comprising: generating a quality score based on the measurement uncertainty.
Clause 28. The method of any of the previous clauses, wherein the measurement uncertainty is reflected in a quality score, wherein higher value quality score indicates higher confidence.
Clause 29. The method of any of the previous clauses, wherein the measurement uncertainty comprises one or more of: direct quantification, optionally comprising a standard deviation; a confidence interval; a likelihood that a measured value is within a specified percent of a true value; or a residual related to a goodness-of-fit of unmixed data.
Clause 30. The method of any of the previous clauses, wherein the measurement uncertainty relates to a binary classification, wherein the binary classification optionally comprises one or more of gating or population membership classifications.
Clause 31. The method of any of the previous clauses, further comprising: calculating a quality score based on the measurement uncertainty,
   wherein the quality score reflects a likelihood of membership in a gate.
Clause 32. The method of clause 31, wherein the likelihood of membership in the gate is calculated for each gate in a gate hierarchy.
Clause 33. The method of clause 31, wherein the likelihood of membership in the gate is calculated taking into account each hierarchical parent gate of the gate.
Clause 34. The method of any of the previous clauses, wherein identifying measurement uncertainty associated with the detected light comprises:
   calculating measurement uncertainty based on a semi-empirical noise model, wherein the semi-empirical noise model comprises one or more of: instrument calibration data, a physical noise model, or measurement values.
Clause 35. The method of any of the previous clauses, wherein identifying measurement uncertainty associated with the detected light comprises:
   estimating measurement uncertainty on a per event basis based on a statistical algorithm, wherein the statistical algorithm comprises measuring properties of data distributions within the sample.
Clause 36. The method of any of the previous clauses, wherein identifying measurement uncertainty associated with the detected light comprises:
   estimating measurement uncertainty comprises applying a combination of noise modeling and distribution fitting.
Clause 37. The method of any of the previous clauses, wherein identifying measurement uncertainty associated with the detected light comprises:
   estimating measurement uncertainty comprises applying Bayesian inference.
Clause 38. The method of any of the previous clauses, further comprising:
   reporting measurement uncertainty for each particle of a subset of particles of the sample.
Clause 39. The method of any of the previous clauses, further comprising:
   reporting measurement uncertainty for a plurality of parameters for each particle of a subset of particles of the sample.
Clause 40. The method of any of the previous clauses, further comprising:
   reporting measurement uncertainty associated with membership in a subpopulation defined by a gate for each particle in a subset of particles.
Clause 41. The method of any of the previous clauses, wherein measurement uncertainty comprises random measurement error.
Clause 42. The method of any of the previous clauses, wherein measurement uncertainty comprises spillover-spreading error.
Clause 43. The method of any of the previous clauses, wherein measurement uncertainty reflects variations other than true intrinsic differences between particles of the sample.
Clause 44. The method of any of the previous clauses, wherein measurement uncertainty reflects confidence in a classification decision regarding particles of the sample.
Clause 45. The method of any of the previous clauses, wherein measurement uncertainty reflects an aggregate measurement uncertainty related to a plurality of parameter measurements of light detected from particles.
Clause 46. The method of any of the previous clauses, wherein measurement uncertainty reflects an aggregate measurement uncertainty related to measurements of light detected from a plurality of particles.
Clause 47. The method of any of the previous clauses, wherein measurement uncertainty reflects an aggregate measurement uncertainty related to the sample.
Clause 48. The method of any of the previous clauses, wherein identifying measurement uncertainty associated with the detected light comprises one or more of:
   estimating measurement uncertainty associated with the detected light,
   measuring measurement uncertainty associated with the detected light, or
   predicting measurement uncertainty associated with the detected light.
Clause 49. The method of any of the previous clauses, further comprising:
   classifying particles based on detected light; and
   calculating a statistical significance for classification of particles based at least in part on the measurement uncertainty.
Clause 50. The method of any of the previous clauses, further comprising:
   classifying particles based on detected light; and
   calculating a confidence interval for classification of particles based at least in part on the measurement uncertainty.
Clause 51. The method of any of the previous clauses, further comprising:
   applying a variance-stabilizing transform to data comprising measurements of light detected from particles in the sample based at least in part on the measurement uncertainty; and
   visualizing aspects of the transformed data.
Clause 52. The method of any of the previous clauses, further comprising:
   preprocessing data comprising measurements of light detected from particles in the sample to minimize intra-cluster variance based at least in part on the measurement uncertainty; and
   applying a clustering algorithm to the preprocessed data, wherein the clustering algorithm optionally comprises one or more of a supervised clustering algorithm and an unsupervised clustering algorithm.
Clause 53. The method of any of the previous clauses, further comprising:
   standardizing data comprising measurements of light detected from particles in the sample to normalize measurement uncertainty across different datasets based at least in part on the measurement uncertainty, wherein the different data sets optionally comprise one or more of datasets collected on different instruments or datasets collected under different conditions.
Clause 54. The method of any of the previous clauses, further comprising:
   using the measurement uncertainty for probabilistic analysis of particle classification or sorting, wherein the probabilistic classification optionally comprises applying a fuzzy logic technique.
Clause 55. The method of any of the previous clauses, wherein the method is a method of distinguishing true variability in the particles of the sample from measurement error.
Clause 56. The method of any of the previous clauses, wherein the method is a method of distinguishing true biological variability from measurement error.
Clause 57. The method of any of the previous clauses, wherein the method is a method of improving sensitivity of flow cytometry assays.
Clause 58. The method of any of the previous clauses, wherein the method is a method of improving resolving power of flow cytometry assays.
Clause 59. The method of any of the previous clauses, wherein the method is a method for assessing a quality of particle analysis results by associating event-specific estimates of measurement uncertainty with flow cytometry measurements.
Clause 60. The method of any of the previous clauses, wherein a quantitative metric of measurement uncertainty is associated with flow cytometry measurement.
Clause 61. The method of any of the previous clauses, further comprising:
   using measurement uncertainty in connection with data analysis or sorting.
Clause 62. The method of any of the previous clauses, further comprising: identifying a gate membership confidence score for each event and each gate, wherein the gate membership confidence score comprises a likelihood that a true biological expression level for a given event falls within a given gate.
Clause 63. The method of any of the previous clauses, wherein the method is a method for calculating gate membership confidence scores.
Clause 64. The method of any of the previous clauses, further comprising:
   using gate membership confidence scores based on measurement uncertainty in particle classification and sorting, wherein particle classification and sorting comprises probabilistic sorting with configurable likelihood thresholds to maximize purity and/or yield.
Clause 65. The method of any of the previous clauses, wherein light is detected with a light detection system.
Clause 66. The method of clause 65, wherein light is detected by the light detection system in a plurality of photodetector channels.
Clause 67. The method of any of clauses 65 to 66, wherein the light detection system comprises a plurality of photodetectors.
Clause 68. A system comprising:
   a light source configured to irradiate a sample comprising a plurality of particles;
   a light detection system comprising a plurality of photodetectors; and
   a processor comprising memory operably coupled to the processor wherein the memory comprises instructions stored thereon, which when executed by the processor, cause the processor to identify measurement uncertainty associated with light detected from the light detection system.
Clause 69. The system of clause 68, wherein light is detected by the light detection system in a plurality of photodetector channels.
Clause 70. The system of any of clauses 68 to 69, wherein the light detection system comprises a plurality of photodetectors.
Clause 71. The system of any of clauses 68 to 70, wherein measurement uncertainty is identified corresponding to individual particles in the sample.
Clause 72. The system of any of clauses 68 to 71, wherein detecting light from particles comprises measuring a plurality of parameters of the detected light for particles in the sample.
Clause 73. The system of any of clauses 68 to 72, wherein measurement uncertainty is identified for individual parameters of detected light for particles in the sample.
Clause 74. The system of any of clauses 68 to 73, wherein the system is further configured to generate event data based on the detected light, wherein event data comprises parameter measurements for particles in the sample.
Clause 75. The system of any of clauses 68 to 74, wherein the memory comprises instructions stored thereon, which when executed by the processor, cause the processor to spectrally resolve the light detected from particles.
Clause 76. The system of any of clauses 68 to 75, wherein spectrally resolving the light detected from particles produces a plurality of derived parameters.
Clause 77. The system of any of any of clauses 68 to 76, wherein the memory comprises instructions stored thereon, which when executed by the processor, cause the processor to generate a plurality of derived parameters for each particle by spectrally resolving the light detected from each particle.
Clause 78. The system of any of clauses 68 to 77, wherein the derived parameters comprise unmixed detected light.
Clause 79. The system of any of clauses 68 to 78, wherein the memory comprises instructions stored thereon, which when executed by the processor, cause the processor to identify measurement uncertainty for derived parameters.
Clause 80. The system of any of clauses 68 to 79, wherein the memory comprises instructions stored thereon, which when executed by the processor, cause the processor to identify a first subset of particles in the sample by applying a first gate to the detected light.
Clause 81. The system of clause 80, wherein measurement uncertainty corresponds to identifying the first subset of particles.
Clause 82. The system of any of clauses 68 to 81, wherein the memory comprises instructions stored thereon, which when executed by the processor, cause the processor to identify a plurality of subsets of particles in the sample by applying a corresponding plurality of gates to the detected light.
Clause 83. The system of clause 82, wherein measurement uncertainty corresponds to identifying each of the plurality of subsets of particles.
Clause 84. The system of any of clauses 68 to 83, wherein measurement uncertainty corresponds to gate-level uncertainty.
Clause 85. The system of any of clauses 68 to 84, wherein the memory comprises instructions stored thereon, which when executed by the processor, cause the processor to generate a quality score (Q score) for each particle based on the measurement uncertainty for each particle.
Clause 86. The system of any of clauses 68 to 85, wherein the memory comprises instructions stored thereon, which when executed by the processor, cause the processor to record each event and associated measurement uncertainty for each event
Clause 87. The system of any of clauses 68 to 86, wherein the memory comprises instructions stored thereon, which when executed by the processor, cause the processor to record measurements for each particle and associated measurement uncertainty for each particle.
Clause 88. The system of any of clauses 68 to 87, wherein the memory comprises instructions stored thereon, which when executed by the processor, cause the processor to record measurements for a plurality of parameters for each particle and associated measurement uncertainty for each parameter of each particle.
Clause 89. The system of any of clauses 68 to 88, wherein the memory comprises instructions stored thereon, which when executed by the processor, cause the processor to record measurement uncertainty, wherein the measurement uncertainty corresponds to measurement uncertainty for one or more of: each recorded parameter; each recorded event; each gate or other classification; or for the sample.
Clause 90. The system of any of clauses 68 to 89, wherein the measurement uncertainty for different parameters measured for particles in the sample comprises a different metric.
Clause 91. The system of any of clauses 68 to 90, wherein the measurement uncertainty corresponds to a binary classification event.
Clause 92. The system of clause 91, wherein the binary classification event corresponds to one or more of: a gating determination or a population membership classification decision.
Clause 93. The system of any of clauses 68 to 92, wherein the measurement uncertainty comprises a metric that quantifies measurement uncertainty.
Clause 94. The system of any of clauses 68 to 93, wherein the memory comprises instructions stored thereon, which when executed by the processor, cause the processor to generate an uncertainty score based on the measurement uncertainty.
Clause 95. The system of any of clauses 68 to 94, wherein measurement uncertainty is reflected in an uncertainty score, wherein higher value uncertainty scores indicates greater measurement uncertainty.
Clause 96. The system of any of clauses 68 to 95, wherein the memory comprises instructions stored thereon, which when executed by the processor, cause the processor to generate a quality score based on the measurement uncertainty.
Clause 97. The system of any of clauses 68 to 96, wherein the measurement uncertainty is reflected in a quality score, wherein higher value quality score indicates higher confidence.
Clause 98. The system of any of clauses 68 to 97, wherein the measurement uncertainty comprises one or more of: direct quantification, optionally comprising a standard deviation; a confidence interval; a likelihood that a measured value is within a specified percent of a true value; or a residual related to a goodness-of-fit of unmixed data.
Clause 99. The system of any of clauses 68 to 98, wherein the measurement uncertainty relates to a binary classification, wherein the binary classification optionally comprises one or more of gating or population membership classifications.
Clause 100. The system of any of clauses 68 to 99, wherein the memory comprises instructions stored thereon, which when executed by the processor, cause the processor to calculate a quality score based on the measurement uncertainty, wherein the quality score reflects a likelihood of membership in a gate.
Clause 101. The system of clause 100, wherein the likelihood of membership in the gate is calculated for each gate in a gate hierarchy.
Clause 102. The system of clause 100, wherein the likelihood of membership in the gate is calculated taking into account each hierarchical parent gate of the gate.
Clause 103. The system of any of clauses 68 to 102, wherein identifying measurement uncertainty associated with the detected light comprises:
   calculating measurement uncertainty based on a semi-empirical noise model, wherein the semi-empirical noise model comprises one or more of: instrument calibration data, a physical noise model, or measurement values.
Clause 104. The system of any of clauses 68 to 103, wherein identifying measurement uncertainty associated with the detected light comprises:
   estimating measurement uncertainty on a per event basis based on a statistical algorithm, wherein the statistical algorithm comprises measuring properties of data distributions within the sample.
Clause 105. The system of any of clauses 68 to 104, wherein identifying measurement uncertainty associated with the detected light comprises:
   estimating measurement uncertainty comprises applying a combination of noise modeling and distribution fitting.
Clause 106. The system of any of clauses 68 to 105, wherein identifying measurement uncertainty associated with the detected light comprises:
   estimating measurement uncertainty comprises applying Bayesian inference. Clause 107. The system of any of clauses 68 to 106, wherein the memory comprises instructions stored thereon, which when executed by the processor, cause the processor to report measurement uncertainty for each particle of a subset of particles of the sample.
Clause 108. The system of any of clauses 68 to 107, wherein the memory comprises instructions stored thereon, which when executed by the processor, cause the processor to report measurement uncertainty for a plurality of parameters for each particle of a subset of particles of the sample.
Clause 109. The system of any of clauses 68 to 108, wherein the memory comprises instructions stored thereon, which when executed by the processor, cause the processor to report measurement uncertainty associated with membership in a subpopulation defined by a gate for each particle in a subset of particles.
Clause 110. The system of any of clauses 68 to 109, wherein measurement uncertainty comprises random measurement error.
Clause 111. The system of any of clauses 68 to 110, wherein measurement uncertainty comprises spillover-spreading error.
Clause 112. The system of any of clauses 68 to 111, wherein measurement uncertainty reflects variations other than true intrinsic differences between particles of the sample.
Clause 113. The system of any of clauses 68 to 112, wherein measurement uncertainty reflects confidence in a classification decision regarding particles of the sample.
Clause 114. The system of any of clauses 68 to 113, wherein measurement uncertainty reflects an aggregate measurement uncertainty related to a plurality of parameter measurements of light detected from particles.
Clause 115. The system of any of clauses 68 to 114, wherein measurement uncertainty reflects an aggregate measurement uncertainty related to measurements of light detected from a plurality of particles.
Clause 116. The system of any of clauses 68 to 115, wherein measurement uncertainty reflects an aggregate measurement uncertainty related to the sample.
Clause 117. The system of any of clauses 68 to 116, wherein identifying measurement uncertainty associated with the detected light comprises one or more of:
   estimating measurement uncertainty associated with the detected light,
   measuring measurement uncertainty associated with the detected light, or
   predicting measurement uncertainty associated with the detected light.
Clause 118. The system of any of clauses 68 to 117, wherein the memory comprises instructions stored thereon, which when executed by the processor, cause the processor to:
   classify particles based on detected light; and
   calculate a statistical significance for classification of particles based at least in part on the measurement uncertainty.
Clause 119. The system of any of clauses 68 to 118, wherein the memory comprises instructions stored thereon, which when executed by the processor, cause the processor to:
   classify particles based on detected light; and
   calculate a confidence interval for classification of particles based at least in part on the measurement uncertainty.
Clause 120. The system of any of clauses 68 to 119, wherein the memory comprises instructions stored thereon, which when executed by the processor, cause the processor to:
   apply a variance-stabilizing transform to data comprising measurements of light detected from particles in the sample based at least in part on the measurement uncertainty; and
   visualize aspects of the transformed data.
Clause 121. The system of any of clauses 68 to 120, wherein the memory comprises instructions stored thereon, which when executed by the processor, cause the processor to:
   preprocess data comprising measurements of light detected from particles in the sample to minimize intra-cluster variance based at least in part on the measurement uncertainty; and
   apply a clustering algorithm to the preprocessed data, wherein the clustering algorithm optionally comprises one or more of a supervised clustering algorithm and an unsupervised clustering algorithm.
Clause 122. The system of any of clauses 68 to 121, wherein the memory comprises instructions stored thereon, which when executed by the processor, cause the processor to:
   standardize data comprising measurements of light detected from particles in the sample to normalize measurement uncertainty across different datasets based at least in part on the measurement uncertainty, wherein the different data sets optionally comprise one or more of datasets collected on different instruments or datasets collected under different conditions.
Clause 123. The system of any of clauses 68 to 122, wherein the memory comprises instructions stored thereon, which when executed by the processor, cause the processor to:
   use the measurement uncertainty for probabilistic analysis of particle classification or sorting, wherein the probabilistic classification optionally comprises applying a fuzzy logic technique.
Clause 124. The system of any of clauses 68 to 123, wherein the system is configured to distinguish true variability in the particles of the sample from measurement error. Clause 125. The system of any of clauses 68 to 124, wherein the system is configured to distinguish true biological variability from measurement error.
Clause 126. The system of any of clauses 68 to 125, wherein the system is configured to distinguish improving sensitivity of flow cytometry assays.
Clause 127. The system of any of clauses 68 to 126, wherein the system is configured to distinguish improving resolving power of flow cytometry assays.
Clause 128. The system of any of clauses 68 to 127, wherein the system is configured to assess a quality of particle analysis results by associating event-specific estimates of measurement uncertainty with flow cytometry measurements.
Clause 129. The system of any of clauses 68 to 128, wherein the system is configured to associate a quantitative metric of measurement uncertainty with flow cytometry measurement.
Clause 130. The system of any of clauses 68 to 129, wherein the system is configured to use measurement uncertainty in connection with data analysis or sorting.
Clause 131. The system of any of clauses 68 to 130, wherein the memory comprises instructions stored thereon, which when executed by the processor, cause the processor to:
   identify a gate membership confidence score for each event and each gate, wherein the gate membership confidence score comprises a likelihood that a true biological expression level for a given event falls within a given gate.
Clause 132. The system of any of clauses 68 to 131, wherein the system is configured to calculate gate membership confidence scores.
Clause 133. The system of any of clauses 68 to 132, wherein the memory comprises instructions stored thereon, which when executed by the processor, cause the processor to:
   use gate membership confidence scores based on measurement uncertainty in particle classification and sorting, wherein particle classification and sorting comprises probabilistic sorting with configurable likelihood thresholds to maximize purity and/or yield.
Clause 134. An integrated circuit programmed to identify measurement uncertainty associated with light detected from particles in a sample by a light detection system.
Clause 135. The integrated circuit according to clause 134, wherein the integrated circuit is a field programmable gate array (FPGA).
Clause 136. The integrated circuit according to clause 134, wherein the integrated circuit is an application specific integrated circuit (ASIC).
Clause 137. The integrated circuit according to clause 134, wherein the integrated circuit is a complex programmable logic device (CPLD).
Clause 138. The integrated circuit of any of clauses 134 to 137, wherein measurement uncertainty is identified corresponding to individual particles in the sample.
Clause 139. The integrated circuit of any of clauses 134 to 138, wherein detecting light from particles comprises measuring a plurality of parameters of the detected light for particles in the sample.
Clause 140. The integrated circuit of any of clauses 134 to 139, wherein measurement uncertainty is identified for individual parameters of detected light for particles in the sample.
Clause 141. The integrated circuit of any of clauses 134 to 140, wherein the integrated circuit is programmed to:
   generate event data based on the detected light, wherein event data comprises parameter measurements for particles in the sample.
Clause 142. The integrated circuit of any of clauses 134 to 141, wherein the integrated circuit is programmed to:
   spectrally resolve the light detected from particles.
Clause 143. The integrated circuit of any of clauses 134 to 142, wherein spectrally resolving the light detected from particles produces a plurality of derived parameters.
Clause 144. The integrated circuit of any of clauses 134 to 143, wherein the integrated circuit is programmed to:
   generate a plurality of derived parameters for each particle by spectrally resolving the light detected from each particle.
Clause 145. The integrated circuit of any of clauses 134 to 144, wherein the derived parameters comprise unmixed detected light.
Clause 146. The integrated circuit of any of clauses 134 to 145, wherein measurement uncertainty is identified for derived parameters.
Clause 147. The integrated circuit of any of clauses 134 to 146, wherein the integrated circuit is programmed to:
   algorithm for identifying a first subset of particles in the sample by applying a first gate to the detected light.
Clause 148. The integrated circuit of clause 147, wherein measurement uncertainty corresponds to identifying the first subset of particles.
Clause 149. The integrated circuit of any of clauses 134 to 148, wherein the integrated circuit is programmed to:
   identify a plurality of subsets of particles in the sample by applying a corresponding plurality of gates to the detected light.
Clause 150. The integrated circuit of clause 149, wherein measurement uncertainty corresponds to identifying each of the plurality of subsets of particles.
Clause 151. The integrated circuit of any of clauses 134 to 150, wherein measurement uncertainty corresponds to gate-level uncertainty.
Clause 152. The integrated circuit of any of clauses 134 to 151, wherein the integrated circuit is programmed to:
   generate a quality score (Q score) for each particle based on the measurement uncertainty for each particle.
Clause 153. The integrated circuit of any of clauses 134 to 152, wherein the integrated circuit is programmed to:
   record each event and associated measurement uncertainty for each event Clause 154. The integrated circuit of any of clauses 134 to 153, wherein the integrated circuit is programmed to:
   record measurements for each particle and associated measurement uncertainty for each particle.
Clause 155. The integrated circuit of any of clauses 134 to 154, wherein the integrated circuit is programmed to:
   record measurements for a plurality of parameters for each particle and associated measurement uncertainty for each parameter of each particle.
Clause 156. The integrated circuit of any of clauses 134 to 155, wherein the integrated circuit is programmed to:
   record measurement uncertainty, wherein the measurement uncertainty corresponds to measurement uncertainty for one or more of: each recorded parameter; each recorded event; each gate or other classification; or for the sample.
Clause 157. The integrated circuit of any of clauses 134 to 156, wherein the measurement uncertainty for different parameters measured for particles in the sample comprises a different metric.
Clause 158. The integrated circuit of any of clauses 134 to 157, wherein the measurement uncertainty corresponds to a binary classification event.
Clause 159. The integrated circuit of clause 158, wherein the binary classification event corresponds to one or more of: a gating determination or a population membership classification decision.
Clause 160. The integrated circuit of any of clauses 134 to 159, wherein the measurement uncertainty comprises a metric that quantifies measurement uncertainty. Clause 161. The integrated circuit of any of clauses 134 to 160, wherein the integrated circuit is programmed to:
   generate an uncertainty score based on the measurement uncertainty.
Clause 162. The integrated circuit of any of clauses 134 to 161, wherein measurement uncertainty is reflected in an uncertainty score, wherein higher value uncertainty scores indicates greater measurement uncertainty.
Clause 163. The integrated circuit of any of clauses 134 to 162, wherein the integrated circuit is programmed to:
   generate a quality score based on the measurement uncertainty.
Clause 164. The integrated circuit of any of clauses 134 to 163, wherein the measurement uncertainty is reflected in a quality score, wherein higher value quality score indicates higher confidence.
Clause 165. The integrated circuit of any of clauses 134 to 164, wherein the measurement uncertainty comprises one or more of: direct quantification, optionally comprising a standard deviation; a confidence interval; a likelihood that a measured value is within a specified percent of a true value; or a residual related to a goodness-of-fit of unmixed data.
Clause 166. The integrated circuit of any of clauses 134 to 165, wherein the measurement uncertainty relates to a binary classification, wherein the binary classification optionally comprises one or more of gating or population membership classifications.
Clause 167. The integrated circuit of any of clauses 134 to 166, wherein the integrated circuit is programmed to:
   algorithm for calculating a quality score based on the measurement uncertainty,
   wherein the quality score reflects a likelihood of membership in a gate.
Clause 168. The integrated circuit of clause 167, wherein the likelihood of membership in the gate is calculated for each gate in a gate hierarchy.
Clause 169. The integrated circuit of clause 167, wherein the likelihood of membership in the gate is calculated taking into account each hierarchical parent gate of the gate.
Clause 170. The integrated circuit of any of clauses 134 to 169, wherein identifying measurement uncertainty associated with the detected light comprises:
   calculating measurement uncertainty based on a semi-empirical noise model, wherein the semi-empirical noise model comprises one or more of: instrument calibration data, a physical noise model, or measurement values.
Clause 171. The integrated circuit of any of clauses 134 to 170, wherein identifying measurement uncertainty associated with the detected light comprises:
   estimating measurement uncertainty on a per event basis based on a statistical algorithm, wherein the statistical algorithm comprises measuring properties of data distributions within the sample.
Clause 172. The integrated circuit of any of clauses 134 to 171, wherein identifying measurement uncertainty associated with the detected light comprises:
   estimating measurement uncertainty comprises applying a combination of noise modeling and distribution fitting.
Clause 173. The integrated circuit of any of clauses 134 to 172, wherein identifying measurement uncertainty associated with the detected light comprises:
   estimating measurement uncertainty comprises applying Bayesian inference.
Clause 174. The integrated circuit of any of clauses 134 to 173, further comprising:
   reporting measurement uncertainty for each particle of a subset of particles of the sample.
Clause 175. The integrated circuit of any of clauses 134 to 174, further comprising:
   reporting measurement uncertainty for a plurality of parameters for each particle of a subset of particles of the sample.
Clause 176. The integrated circuit of any of clauses 134 to 175, further comprising:
   reporting measurement uncertainty associated with membership in a subpopulation defined by a gate for each particle in a subset of particles.
Clause 177. The integrated circuit of any of clauses 134 to 176, wherein measurement uncertainty comprises random measurement error.
Clause 178. The integrated circuit of any of clauses 134 to 177, wherein measurement uncertainty comprises spillover-spreading error.
Clause 179. The integrated circuit of any of clauses 134 to 178, wherein measurement uncertainty reflects variations other than true intrinsic differences between particles of the sample.
Clause 180. The integrated circuit of any of clauses 134 to 179, wherein measurement uncertainty reflects confidence in a classification decision regarding particles of the sample.
Clause 181. The integrated circuit of any of clauses 134 to 180, wherein measurement uncertainty reflects an aggregate measurement uncertainty related to a plurality of parameter measurements of light detected from particles.
Clause 182. The integrated circuit of any of clauses 134 to 181, wherein measurement uncertainty reflects an aggregate measurement uncertainty related to measurements of light detected from a plurality of particles.
Clause 183. The integrated circuit of any of clauses 134 to 182, wherein measurement uncertainty reflects an aggregate measurement uncertainty related to the sample.
Clause 184. The integrated circuit of any of clauses 134 to 183, wherein identifying measurement uncertainty associated with the detected light comprises one or more of:
   estimating measurement uncertainty associated with the detected light,
   measuring measurement uncertainty associated with the detected light, or
   predicting measurement uncertainty associated with the detected light.
Clause 185. The integrated circuit of any of clauses 134 to 184, wherein the integrated circuit is programmed to:
   classify particles based on detected light; and
   calculate a statistical significance for classification of particles based at least in part on the measurement uncertainty.
Clause 186. The integrated circuit of any of clauses 134 to 185, wherein the integrated circuit is programmed to:
   classify particles based on detected light; and
   calculate a confidence interval for classification of particles based at least in part on the measurement uncertainty.
Clause 187. The integrated circuit of any of clauses 134 to 186, wherein the integrated circuit is programmed to:
   apply a variance-stabilizing transform to data comprising measurements of light detected from particles in the sample based at least in part on the measurement uncertainty; and
   visualize aspects of the transformed data.
Clause 188. The integrated circuit of any of clauses 134 to 188, wherein the integrated circuit is programmed to:
   preprocess data comprising measurements of light detected from particles in the sample to minimize intra-cluster variance based at least in part on the measurement uncertainty;
   apply a clustering algorithm to the preprocessed data, wherein the clustering algorithm optionally comprises one or more of a supervised clustering algorithm and an unsupervised clustering algorithm.
Clause 189. The integrated circuit of any of clauses 134 to 188, wherein the integrated circuit is programmed to:
   standardize data comprising measurements of light detected from particles in the sample to normalize measurement uncertainty across different datasets based at least in part on the measurement uncertainty, wherein the different data sets optionally comprise one or more of datasets collected on different instruments or datasets collected under different conditions.
Clause 190. The integrated circuit of any of clauses 134 to 189, wherein the integrated circuit is programmed to:
   use the measurement uncertainty for probabilistic analysis of particle classification or sorting, wherein the probabilistic classification optionally comprises applying a fuzzy logic technique.
Clause 191. The integrated circuit of any of clauses 134 to 190, wherein the integrated circuit is configured to distinguish true variability in the particles of the sample from measurement error.
Clause 192. The integrated circuit of any of clauses 134 to 191, wherein the integrated circuit is configured to distinguish true biological variability from measurement error.
Clause 193. The integrated circuit of any of clauses 134 to 192, wherein the integrated circuit is configured to distinguish improving sensitivity of flow cytometry assays.
Clause 194. The integrated circuit of any of clauses 134 to 193, wherein the integrated circuit is configured to distinguish improving resolving power of flow cytometry assays.
Clause 195. The integrated circuit of any of clauses 134 to 194, wherein the integrated circuit is configured to assess a quality of particle analysis results by associating event-specific estimates of measurement uncertainty with flow cytometry measurements.
Clause 196. The integrated circuit of any of clauses 134 to 195, wherein a quantitative metric of measurement uncertainty is associated with flow cytometry measurement.
Clause 197. The integrated circuit of any of clauses 134 to 196, wherein the integrated circuit is programmed to:
   use measurement uncertainty in connection with data analysis or sorting.
Clause 198. The integrated circuit of any of clauses 134 to 197, wherein the integrated circuit is programmed to:
   Identify a gate membership confidence score for each event and each gate, wherein the gate membership confidence score comprises a likelihood that a true biological expression level for a given event falls within a given gate.
Clause 199. The integrated circuit of any of clauses 134 to 198, wherein the integrated circuit is configured to calculate gate membership confidence scores.
Clause 200. The integrated circuit of any of clauses 134 to 199, wherein the integrated circuit is programmed to:
   use gate membership confidence scores based on measurement uncertainty in particle classification and sorting, wherein particle classification and sorting comprises probabilistic sorting with configurable likelihood thresholds to maximize purity and/or yield.
Clause 201. A non-transitory computer readable storage medium comprising instructions stored thereon, the instructions comprising:
   algorithm for identifying measurement uncertainty associated with light detected from particles in a sample by a light detection system.
Clause 202. The non-transitory computer readable storage medium of clause 201, wherein measurement uncertainty is identified corresponding to individual particles in the sample.
Clause 203. The non-transitory computer readable storage medium of any of clauses 201 to 202, wherein detecting light from particles comprises measuring a plurality of parameters of the detected light for particles in the sample.
Clause 204. The non-transitory computer readable storage medium of any of clauses 201 to 203, wherein measurement uncertainty is identified for individual parameters of detected light for particles in the sample.
Clause 205. The non-transitory computer readable storage medium of any of clauses 201 to 204, further comprising:
   algorithm for generating event data based on the detected light, wherein event data comprises parameter measurements for particles in the sample.
Clause 206. The non-transitory computer readable storage medium of any of clauses 201 to 205, further comprising:
   algorithm for spectrally resolving the light detected from particles.
Clause 207. The non-transitory computer readable storage medium of any of clauses 201 to 206, wherein spectrally resolving the light detected from particles produces a plurality of derived parameters.
Clause 208. The non-transitory computer readable storage medium of any of clauses 201 to 207, further comprising:
   algorithm for generating a plurality of derived parameters for each particle by spectrally resolving the light detected from each particle.
Clause 209. The non-transitory computer readable storage medium of any of clauses 201 to 208, wherein the derived parameters comprise unmixed detected light.
Clause 210. The non-transitory computer readable storage medium of any of clauses 201 to 209, wherein measurement uncertainty is identified for derived parameters.
Clause 211. The non-transitory computer readable storage medium of any of clauses 201 to 210, further comprising:
   algorithm for identifying a first subset of particles in the sample by applying a first gate to the detected light.
Clause 212. The non-transitory computer readable storage medium of clause 211, wherein measurement uncertainty corresponds to identifying the first subset of particles.
Clause 213. The non-transitory computer readable storage medium of any of clauses 201 to 212, further comprising:
   algorithm for identifying a plurality of subsets of particles in the sample by applying a corresponding plurality of gates to the detected light.
Clause 214. The non-transitory computer readable storage medium of clause 213, wherein measurement uncertainty corresponds to identifying each of the plurality of subsets of particles.
Clause 215. The non-transitory computer readable storage medium of any of clauses 201 to 214, wherein measurement uncertainty corresponds to gate-level uncertainty.
Clause 216. The non-transitory computer readable storage medium of any of clauses 201 to 215, further comprising:
   algorithm for generating a quality score (Q score) for each particle based on the measurement uncertainty for each particle.
Clause 217. The non-transitory computer readable storage medium of any of clauses 201 to 216, further comprising:
   algorithm for recording each event and associated measurement uncertainty for each event
Clause 218. The non-transitory computer readable storage medium of any of clauses 201 to 217, further comprising:
   algorithm for recording measurements for each particle and associated measurement uncertainty for each particle.
Clause 219. The non-transitory computer readable storage medium of any of clauses 201 to 218, further comprising:
   algorithm for recording measurements for a plurality of parameters for each particle and associated measurement uncertainty for each parameter of each particle. Clause 220. The non-transitory computer readable storage medium of any of clauses 201 to 219, further comprising:
   algorithm for recording measurement uncertainty, wherein the measurement uncertainty corresponds to measurement uncertainty for one or more of: each recorded parameter; each recorded event; each gate or other classification; or for the sample.
Clause 221. The non-transitory computer readable storage medium of any of clauses 201 to 220, wherein the measurement uncertainty for different parameters measured for particles in the sample comprises a different metric.
Clause 222. The non-transitory computer readable storage medium of any of clauses 201 to 221, wherein the measurement uncertainty corresponds to a binary classification event.
Clause 223. The non-transitory computer readable storage medium of clause 222, wherein the binary classification event corresponds to one or more of: a gating determination or a population membership classification decision.
Clause 224. The non-transitory computer readable storage medium of any of clauses 201 to 223, wherein the measurement uncertainty comprises a metric that quantifies measurement uncertainty.
Clause 225. The non-transitory computer readable storage medium of any of clauses 201 to 224, further comprising:
   algorithm for generating an uncertainty score based on the measurement uncertainty.
Clause 226. The non-transitory computer readable storage medium of any of clauses 201 to 225, wherein measurement uncertainty is reflected in an uncertainty score, wherein higher value uncertainty scores indicates greater measurement uncertainty.
Clause 227. The non-transitory computer readable storage medium of any of clauses 201 to 226, further comprising:
   algorithm for generating a quality score based on the measurement uncertainty.
Clause 228. The non-transitory computer readable storage medium of any of clauses 201 to 227, wherein the measurement uncertainty is reflected in a quality score, wherein higher value quality score indicates higher confidence.
Clause 229. The non-transitory computer readable storage medium of any of clauses 201 to 228, wherein the measurement uncertainty comprises one or more of: direct quantification, optionally comprising a standard deviation; a confidence interval; a likelihood that a measured value is within a specified percent of a true value; or a residual related to a goodness-of-fit of unmixed data.
Clause 230. The non-transitory computer readable storage medium of any of clauses 201 to 229, wherein the measurement uncertainty relates to a binary classification, wherein the binary classification optionally comprises one or more of gating or population membership classifications.
Clause 231. The non-transitory computer readable storage medium of any of clauses 201 to 230, further comprising:
   algorithm for calculating a quality score based on the measurement uncertainty, wherein the quality score reflects a likelihood of membership in a gate.
Clause 232. The non-transitory computer readable storage medium of clause 231, wherein the likelihood of membership in the gate is calculated for each gate in a gate hierarchy.
Clause 233. The non-transitory computer readable storage medium of clause 231, wherein the likelihood of membership in the gate is calculated taking into account each hierarchical parent gate of the gate.
Clause 234. The non-transitory computer readable storage medium of any of clauses 201 to 233, wherein identifying measurement uncertainty associated with the detected light comprises:
   algorithm for calculating measurement uncertainty based on a semi-empirical noise model, wherein the semi-empirical noise model comprises one or more of:
   instrument calibration data, a physical noise model, or measurement values.
Clause 235. The non-transitory computer readable storage medium of any of clauses 201 to 234, wherein identifying measurement uncertainty associated with the detected light comprises:
   algorithm for estimating measurement uncertainty on a per event basis based on a statistical algorithm, wherein the statistical algorithm comprises measuring properties of data distributions within the sample.
Clause 236. The non-transitory computer readable storage medium of any of clauses 201 to 235, wherein identifying measurement uncertainty associated with the detected light comprises:
   algorithm for estimating measurement uncertainty comprises applying a combination of noise modeling and distribution fitting.
Clause 237. The non-transitory computer readable storage medium of any of clauses 201 to 236, wherein identifying measurement uncertainty associated with the detected light comprises:
   algorithm for estimating measurement uncertainty comprises applying Bayesian inference.
Clause 238. The non-transitory computer readable storage medium of any of clauses 201 to 237, further comprising:
   algorithm for reporting measurement uncertainty for each particle of a subset of particles of the sample.
Clause 239. The non-transitory computer readable storage medium of any of clauses 201 to 238, further comprising:
   algorithm for reporting measurement uncertainty for a plurality of parameters for each particle of a subset of particles of the sample.
Clause 240. The non-transitory computer readable storage medium of any of clauses 201 to 239, further comprising:
   algorithm for reporting measurement uncertainty associated with membership in a subpopulation defined by a gate for each particle in a subset of particles.
Clause 241. The non-transitory computer readable storage medium of any of clauses 201 to 240, wherein measurement uncertainty comprises random measurement error.
Clause 242. The non-transitory computer readable storage medium of any of clauses 201 to 241, wherein measurement uncertainty comprises spillover-spreading error.
Clause 243. The non-transitory computer readable storage medium of any of clauses 201 to 242, wherein measurement uncertainty reflects variations other than true intrinsic differences between particles of the sample.
Clause 244. The non-transitory computer readable storage medium of any of clauses 201 to 243, wherein measurement uncertainty reflects confidence in a classification decision regarding particles of the sample.
Clause 245. The non-transitory computer readable storage medium of any of clauses 201 to 244, wherein measurement uncertainty reflects an aggregate measurement uncertainty related to a plurality of parameter measurements of light detected from particles.
Clause 246. The non-transitory computer readable storage medium of any of clauses 201 to 245, wherein measurement uncertainty reflects an aggregate measurement uncertainty related to measurements of light detected from a plurality of particles. Clause 247. The non-transitory computer readable storage medium of any of clauses 201 to 246, wherein measurement uncertainty reflects an aggregate measurement uncertainty related to the sample.
Clause 248. The non-transitory computer readable storage medium of any of clauses 201 to 247, wherein identifying measurement uncertainty associated with the detected light comprises one or more of:
   estimating measurement uncertainty associated with the detected light,
   measuring measurement uncertainty associated with the detected light, or
   predicting measurement uncertainty associated with the detected light.
Clause 249. The non-transitory computer readable storage medium of any of clauses 201 to 248, further comprising:
   algorithm for classifying particles based on detected light; and
   algorithm for calculating a statistical significance for classification of particles based at least in part on the measurement uncertainty.
Clause 250. The non-transitory computer readable storage medium of any of clauses 201 to 249, further comprising:
   algorithm for classifying particles based on detected light; and
   algorithm for calculating a confidence interval for classification of particles based at least in part on the measurement uncertainty.
Clause 251. The non-transitory computer readable storage medium of any of clauses 201 to 250, further comprising:
   algorithm for applying a variance-stabilizing transform to data comprising measurements of light detected from particles in the sample based at least in part on the measurement uncertainty; and
   algorithm for visualizing aspects of the transformed data.
Clause 252. The non-transitory computer readable storage medium of any of clauses 201 to 251, further comprising:
   algorithm for preprocessing data comprising measurements of light detected from particles in the sample to minimize intra-cluster variance based at least in part on the measurement uncertainty;
   algorithm for applying a clustering algorithm to the preprocessed data, wherein the clustering algorithm optionally comprises one or more of a supervised clustering algorithm and an unsupervised clustering algorithm.
Clause 253. The non-transitory computer readable storage medium of any of clauses 201 to 252, further comprising:
   algorithm for standardizing data comprising measurements of light detected from particles in the sample to normalize measurement uncertainty across different datasets based at least in part on the measurement uncertainty, wherein the different data sets optionally comprise one or more of datasets collected on different instruments or datasets collected under different conditions.
Clause 254. The non-transitory computer readable storage medium of any of clauses 201 to 253, further comprising:
   algorithm for using the measurement uncertainty for probabilistic analysis of particle classification or sorting, wherein the probabilistic classification optionally comprises applying a fuzzy logic technique.
Clause 255. The non-transitory computer readable storage medium of any of clauses 201 to 254, wherein a quantitative metric of measurement uncertainty is associated with flow cytometry measurement.
Clause 256. The non-transitory computer readable storage medium of any of clauses 201 to 255, further comprising:
   algorithm for using measurement uncertainty in connection with data analysis or sorting.
Clause 257. The non-transitory computer readable storage medium of any of clauses 201 to 256, further comprising:
   algorithm for identifying a gate membership confidence score for each event and each gate, wherein the gate membership confidence score comprises a likelihood that a true biological expression level for a given event falls within a given gate.
Clause 258. The non-transitory computer readable storage medium of any of clauses 201 to 257, wherein the method is a method for calculating gate membership confidence scores.
Clause 259. The non-transitory computer readable storage medium of any of clauses 201 to 258, further comprising:
   algorithm for using gate membership confidence scores based on measurement uncertainty in particle classification and sorting, wherein particle classification and sorting comprises probabilistic sorting with configurable likelihood thresholds to maximize purity and/or yield.

Although the foregoing disclosure has been described in some detail by way of illustration and example for purposes of clarity of understanding, it is readily apparent to those of ordinary skill in the art in light of the teachings of this disclosure that some changes and modifications may be made thereto without departing from the scope of the appended claims.

Accordingly, the preceding merely illustrates the principles of the disclosure. It will be appreciated that those skilled in the art will be able to devise various arrangements which, although not explicitly described or shown herein, embody the principles of the disclosure and are included within its scope. Furthermore, all examples and conditional language recited herein are principally intended to aid the reader in understanding the principles of the disclosure and the concepts contributed by the inventors to furthering the art, and are to be construed as being without limitation to such specifically recited examples and conditions. Moreover, all statements herein reciting principles, aspects, and embodiments of the disclosure as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. Moreover, nothing disclosed herein is intended to be dedicated to the public regardless of whether such disclosure is explicitly recited in the claims.

## Claims

1. A method comprising:
introducing a sample into a flow cytometer;
flowing the introduced sample in a flow stream;
irradiating the sample in the flow stream with a light source;
detecting light from particles in the sample flowing in the flow stream; and
identifying measurement uncertainty associated with the detected light.

2. The method of claim 1, wherein the measurement uncertainty relates to a binary classification, wherein the binary classification optionally comprises one or more of gating or population membership classifications.

3. The method of claims 1 or 2, further comprising:
calculating a quality score based on the measurement uncertainty,
wherein the quality score reflects a likelihood of membership in a gate, preferably wherein the likelihood of membership in the gate is calculated for each gate in a gate hierarchy, and/or wherein the likelihood of membership in the gate is calculated taking into account each hierarchical parent gate of the gate.

4. The method of any of the previous claims, wherein identifying measurement uncertainty associated with the detected light comprises one or more of:
estimating measurement uncertainty associated with the detected light,
measuring measurement uncertainty associated with the detected light, or
predicting measurement uncertainty associated with the detected light.

5. The method of any of the previous claims, further comprising:
classifying particles based on detected light; and
calculating a confidence interval for classification of particles based at least in part on the measurement uncertainty, and/or
further comprising:
using the measurement uncertainty for probabilistic analysis of particle classification or sorting, wherein the probabilistic classification optionally comprises applying a fuzzy logic technique.

6. The method of any of the previous claims, further comprising:
identifying a gate membership confidence score for each event and each gate, wherein the gate membership confidence score comprises a likelihood that a true biological expression level for a given event falls within a given gate, and/or wherein the method is a method for calculating gate membership confidence scores.

7. The method of any of the previous claims, further comprising:
using gate membership confidence scores based on measurement uncertainty in particle classification and sorting, wherein particle classification and sorting comprises probabilistic sorting with configurable likelihood thresholds to maximize purity and/or yield.

8. A system comprising:
a light source configured to irradiate a sample comprising a plurality of particles;
a light detection system comprising a plurality of photodetectors; and
a processor comprising memory operably coupled to the processor wherein the memory comprises instructions stored thereon, which when executed by the processor, cause the processor to identify measurement uncertainty associated with light detected from the light detection system.

9. The system of claim 8, wherein the measurement uncertainty relates to a binary classification, wherein the binary classification optionally comprises one or more of gating or population membership classifications.

10. The system of any of claims 8 or 9, wherein the memory comprises instructions stored thereon, which when executed by the processor, cause the processor to calculate a quality score based on the measurement uncertainty, wherein the quality score reflects a likelihood of membership in a gate, preferably wherein the likelihood of membership in the gate is calculated for each gate in a gate hierarchy, and/or wherein the likelihood of membership in the gate is calculated taking into account each hierarchical parent gate of the gate.

11. The system of any of claims 8 to 10, wherein identifying measurement uncertainty associated with the detected light comprises one or more of:
estimating measurement uncertainty associated with the detected light,
measuring measurement uncertainty associated with the detected light, or
predicting measurement uncertainty associated with the detected light.

12. The system of any of claims 8 to 11, wherein the memory comprises instructions stored thereon, which when executed by the processor, cause the processor to:
classify particles based on detected light; and
calculate a confidence interval for classification of particles based at least in part on the measurement uncertainty.

13. The system of any of claims 8 to 12, wherein the memory comprises instructions stored thereon, which when executed by the processor, cause the processor to:
use the measurement uncertainty for probabilistic analysis of particle classification or sorting, wherein the probabilistic classification optionally comprises applying a fuzzy logic technique.

14. The system of any of claims 11 to 13, wherein the memory comprises instructions stored thereon, which when executed by the processor, cause the processor to:
identify a gate membership confidence score for each event and each gate, wherein the gate membership confidence score comprises a likelihood that a true biological expression level for a given event falls within a given gate, and/or wherein the system is configured to calculate gate membership confidence scores.

15. The system of any of claims 8 to 14, wherein the memory comprises instructions stored thereon, which when executed by the processor, cause the processor to:
use gate membership confidence scores based on measurement uncertainty in particle classification and sorting, wherein particle classification and sorting comprises probabilistic sorting with configurable likelihood thresholds to maximize purity and/or yield.
